# EUROPEAN PATENT APPLICATION

(11) **EP 4 166 287 A1**
(43) Date of publication of application: **19.04.2023**
(21) Application number: 21821335.3
(22) Date of filing: 11.06.2021
(51) Int. Cl.: B25J 17/00, A61F 2/64, A61F 2/70

(54) **JOINT DEVICE**

(30) Priority: 12.06.2020 JP 2020102714
(71) Applicant: HONDA MOTOR CO., LTD., Minato-ku Tokyo 107-8556 (JP)
(72) Inventor: ONO, Hiromi, Wako-shi, Saitama 351-0193 (JP); SHIMADA, Kei, Wako-shi, Saitama 351-0193 (JP)
(74) Representative: Herzog, Markus
(86) International application number: PCT/JP2021/022406
(87) International publication number: WO 2021/251500

(57) **Abstract**

A motorized leg (1) is provided with a lower knee member (110), an upper knee member (120), a knee joint mechanism (130) coupling the lower knee member (110) and the upper knee member (120) such that the angle therebetween can be changed, and an extendable device (140) capable of changing the angle between the lower knee member (110) and the upper knee member (120) by extending and contracting. The extendable device (140) comprises a motor (M) and a transmission (T) that transmits power from the motor (M). The transmission (T) comprises a first transmission mechanism (T1) that transmits power from the motor (M) at a first gear ratio, and a second transmission mechanism (T2) that transmits power from the motor (M) at a second gear ratio different from the first gear ratio.

## Description

### TECHNICAL FIELD

The present invention relates to a joint device.

### BACKGROUND ART

In the related art, as a joint device used in a connecting unit that connects two members, there is a joint device including an expansion and contraction device capable of changing an angle formed by the two members. As such a joint device, for example, there is a prosthetic leg used for a knee joint. Patent Literature 1 discloses that a sensor for detecting a contraction motion of muscles at a cut end portion of a cut leg is provided in a thigh socket of a prosthetic leg attached to the cut end portion of the cut leg, and a throttle degree of a variable valve of a hydraulic cylinder for adjusting resistance to flexion and extension of a knee joint portion is controlled based on detection information from the sensor.

### CITATION LIST

### PATENT LITERATURE

Patent Literature 1: JPH11-19105A

### SUMMARY OF INVENTION

### TECHNICAL PROBLEM

However, the prosthetic leg disclosed in Patent Literature 1 can generate the resistance to flexion and extension, but cannot generate a power for flexion and extension. In particular, in order to go up stairs smoothly, it is necessary to extend the knee joint while a load is applied.

The present invention provides a joint device capable of extending and flexing a connecting unit by a power of a power source.

### SOLUTION TO PROBLEM

The present invention relates to a joint device including:
a first member;
a second member;
a connecting unit connecting the first member and the second member such that an angle formed by the first member and the second member is changeable; and
an expansion and contraction device configured to change the angle formed by the first member and the second member by expansion and contraction, in which
the expansion and contraction device includes:
   a power source; and
   a power transmission unit configured to transmit power of the power source, and
the power transmission unit includes:
   a first power transmission path through which the power is transmitted at a first transmission gear ratio; and
   a second power transmission path through which the power is transmitted at a second transmission gear ratio different from the first transmission gear ratio.

### ADVANTAGEOUS EFFECTS OF INVENTION

According to the present invention, the connecting unit can be extended and flexed via the power transmission unit that transmits the power of the power source.

### BRIEF DESCRIPTION OF DRAWINGS

Fig. 1 is a perspective view of an electric prosthetic leg according to a first embodiment of the present invention as viewed obliquely from a front.
Fig. 2 is a diagram showing a power transmission unit of the electric prosthetic leg in Fig. 1.
Fig. 3 is a diagram showing a first shift state in which a first connection and disconnection portion of a first connection and disconnection mechanism is in a forced free state and a third connection and disconnection portion of a second connection and disconnection mechanism is in a power transmissible state in the power transmission unit in Fig. 2.
Fig. 4 is a diagram showing a second shift state in which the first connection and disconnection portion of the first connection and disconnection mechanism is in the power transmissible state and the third connection and disconnection portion of the second connection and disconnection mechanism is in the forced free state in the power transmission unit in Fig. 2.
Fig. 5 is a cross-sectional view taken along a line A-A in Fig. 3.
Fig. 6 is a cross-sectional view taken along a line B-B in Fig. 3.
Fig. 7 is a perspective view taken along a section C-C in Fig. 3.
Fig. 8 is a perspective view taken along a section D-D in Fig. 3.
Parts (A) to (F) of Fig. 9 show motions of a human and the electric prosthetic leg when going up stairs.
Fig. 10 is a diagram illustrating a power when a knee joint mechanism is extended from a flexed state when going up the stairs (part (A) → part (B) of Fig. 9 ).
Fig. 11 is a diagram illustrating a power when the knee joint mechanism is flexed from an extension state when going up the stairs (part (D) → part (E) of Fig. 9 ).
Fig. 12 is a diagram showing motions of the human and the electric prosthetic leg when going up the stairs, walking on a flat ground, and going down the stairs.
Fig. 13 is a diagram illustrating a power when the knee joint mechanism is flexed from the extension state while an external force is damped when going down the stairs or walking on the flat ground.
Fig. 14 is a diagram showing an electric prosthetic leg according to a modification, and is a diagram illustrating a power when the knee joint mechanism is extended from the flexed state while the external force is damped when walking on the flat ground.
Fig. 15 is a diagram showing a power transmission unit of an electric prosthetic leg according to a second embodiment of the present invention.
Fig. 16 is a diagram showing a power transmission unit of an electric prosthetic leg according to a third embodiment of the present invention.
Fig. 17 is a cross-sectional view of a two-way clutch.
Fig. 18 is a perspective view showing an example of a retainer (including rollers, guides, and rubber bulbs) shown in Fig. 17.
Fig. 19 is a perspective view showing another example of the retainer (including the rollers, the guides, and the O-rings) shown in Fig. 17.

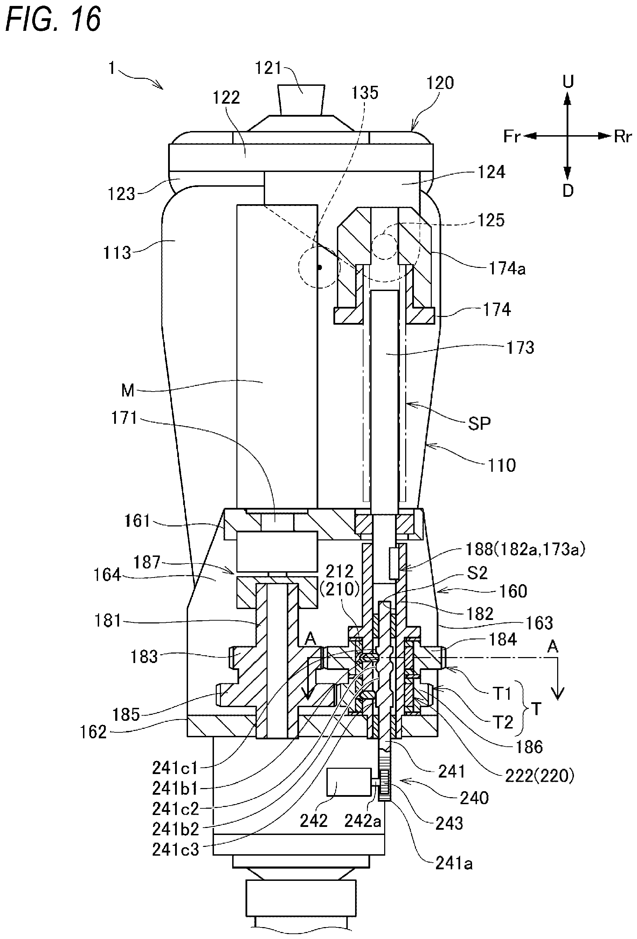

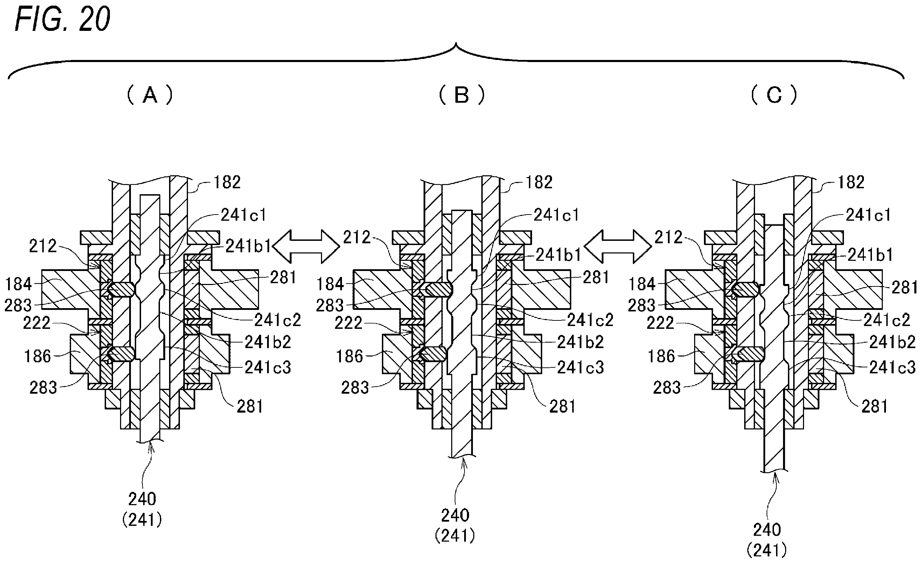

Fig. 20 is a diagram showing an operation of a second operation mechanism 240 in a second connection and disconnection portion and a fourth connection and disconnection portion shown in Fig. 16, part (A) of Fig. 20 shows that the second connection and disconnection portion and the fourth connection and disconnection portion are in an OFF state, part (B) of Fig. 20 shows that the second connection and disconnection portion is in an ON state and the fourth connection and disconnection portion is in the OFF state, and part (C) of Fig. 20 shows that the second connection and disconnection portion is in the OFF state and the fourth connection and disconnection portion in the ON state.
Part (A) of Fig. 21 is a cross-sectional view taken along a position A-A in Fig. 16 showing that the second connection and disconnection portion is in the OFF state, and part (B) of Fig. 21 shows a position of a second operation rod 241 in that case.
Part (A) of Fig. 22 is a cross-sectional view taken along the position A-A in Fig. 16 showing that the second connection and disconnection portion is in the ON state from the OFF state, and part (B) of Fig. 22 shows the position of the second operation rod 241 in that case.
Part (A) of Fig. 23 is a cross-sectional view taken along the position A-A in Fig. 16 showing s forward rotation ON state of the second connection and disconnection portion shown in Fig. 16, and part (B) of Fig. 23 shows the position of the second operation rod 241 in that case.
Part (A) of Fig. 24 is a cross-sectional view taken along the line A-A in Fig. 16 showing a reverse rotation OFF state of the second connection and disconnection portion shown in Fig. 16, and part (B) of Fig. 24 shows the position of the second operation rod 241 in that case.
Part (A) of Fig. 25 is a cross-sectional view taken along the position A-A in Fig. 16 showing the forward rotation ON state of the second connection and disconnection portion shown in Fig. 16, and part (B) of Fig. 25 shows the position of the second operation rod 241 in that case.
Part (A) of Fig. 26 is a cross-sectional view taken along the position A-A in Fig. 16 showing that the second connection and disconnection portion shown in Fig. 16 is in the ON state from the OFF state, and part (B) of Fig. 26 shows the position of the second operation rod 241 in that case.

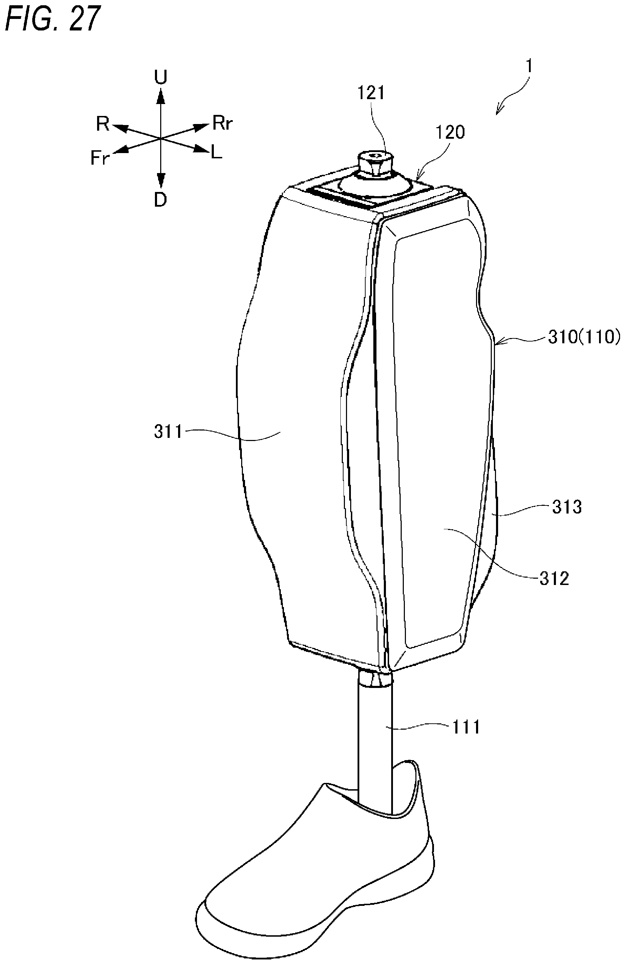

Fig. 27 is a perspective view of an electric prosthetic leg according to a fourth embodiment of the present invention as viewed obliquely from a front.

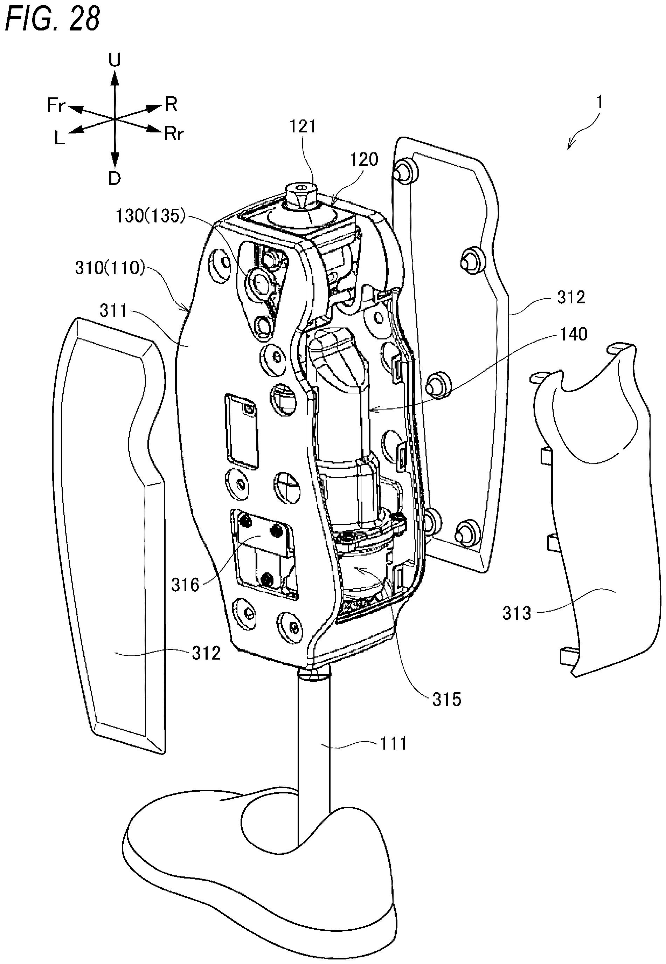

Fig. 28 is an exploded perspective view of the electric prosthetic leg in Fig. 27.

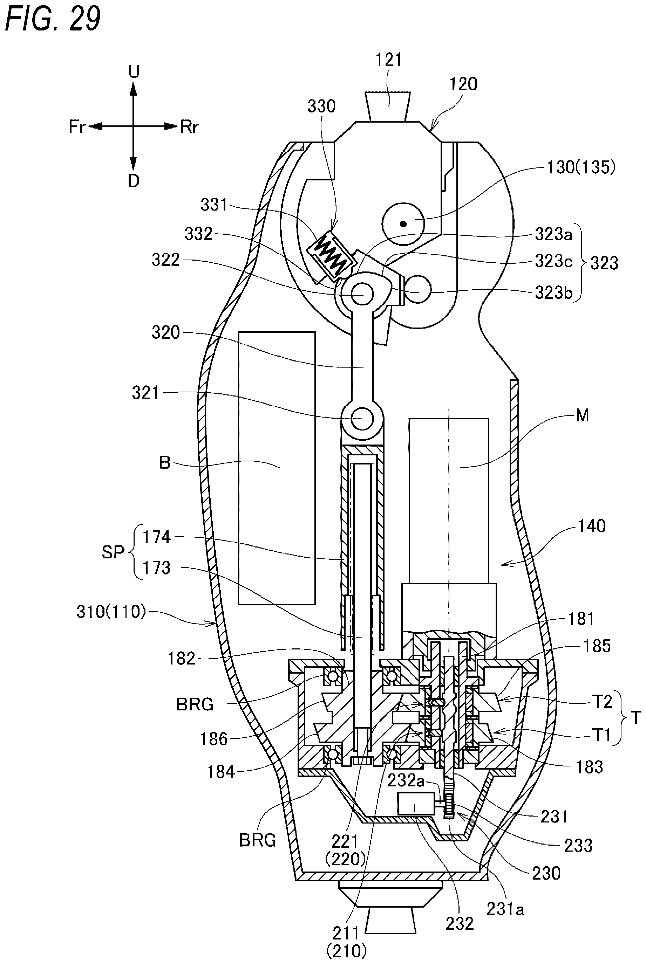

Fig. 29 is a cross-sectional view of the electric prosthetic leg in Fig. 27.

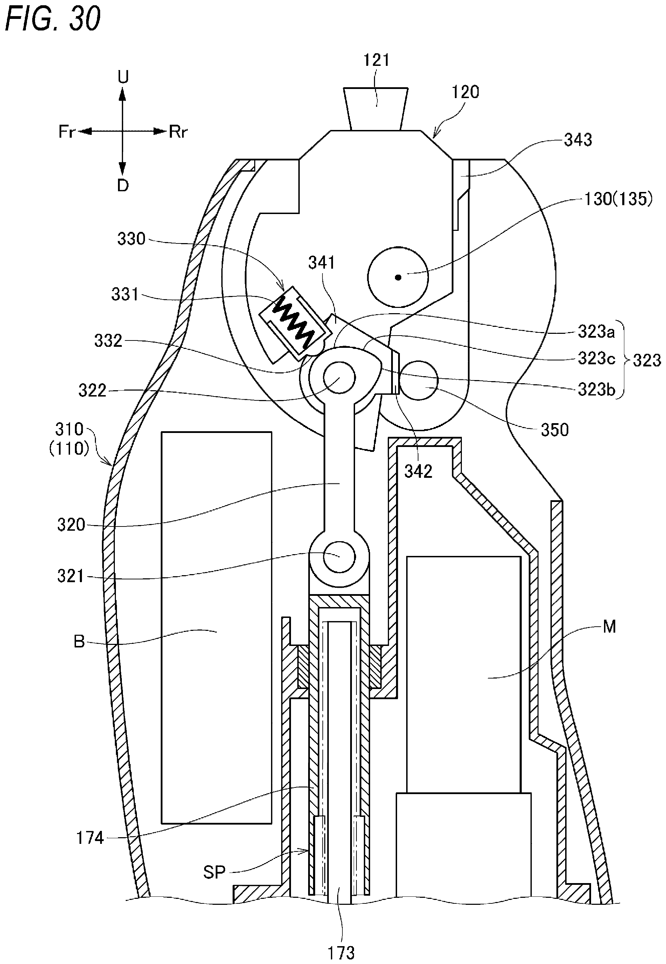

Fig. 30 is a cross-sectional view of a main part showing an extension state of the electric prosthetic leg in Fig. 27.

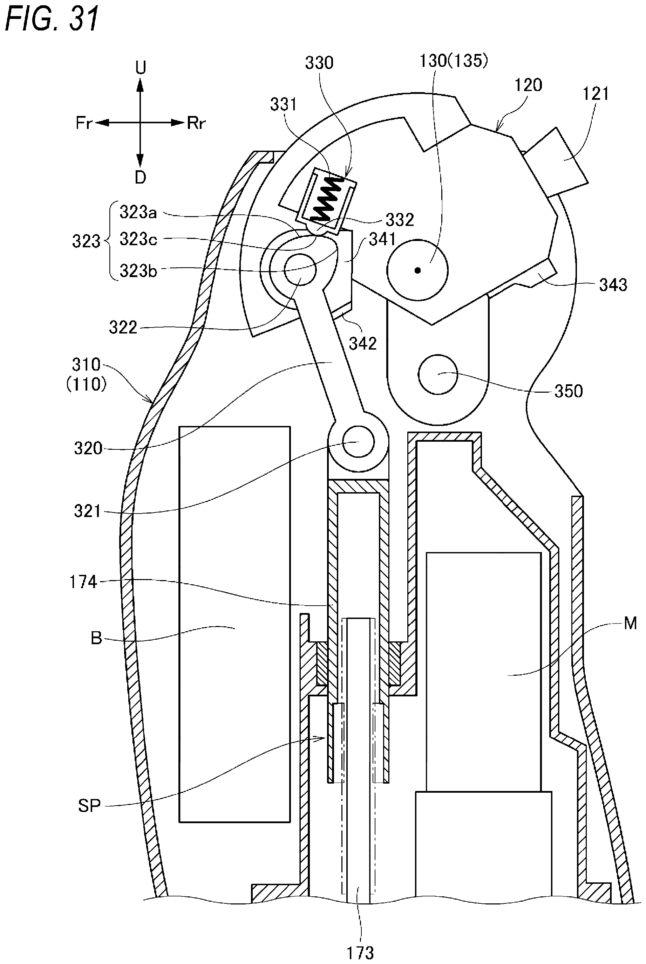

Fig. 31 is a cross-sectional view of a main part showing the electric prosthetic leg in Fig. 27 during flexing.

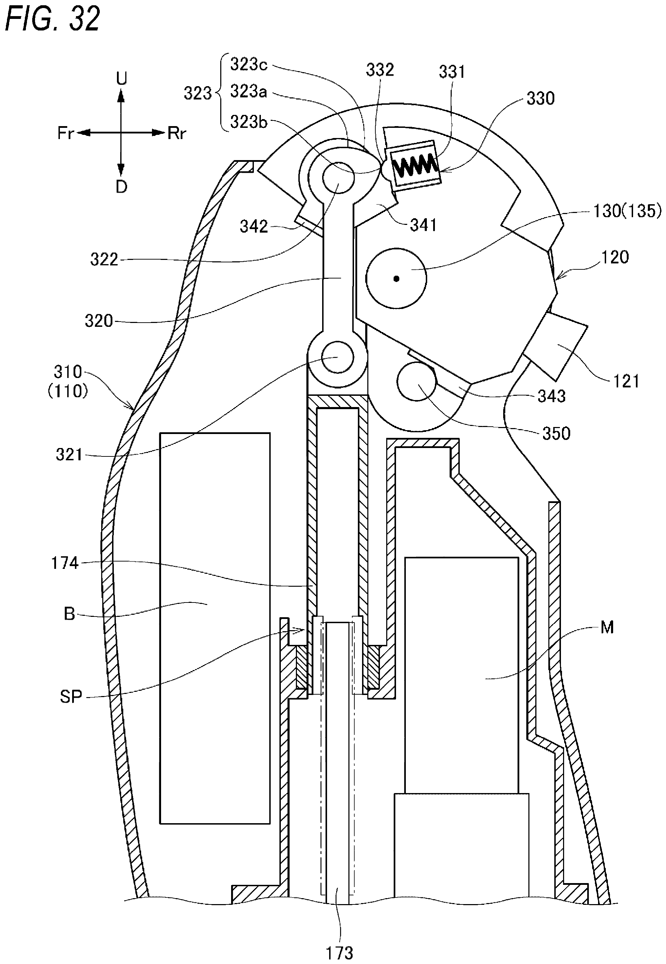

Fig. 32 is a cross-sectional view of a main part showing a maximum flexion state of the electric prosthetic leg in Fig. 27.

### DESCRIPTION OF EMBODIMENTS

Hereinafter, embodiments of an electric prosthetic leg as examples of a joint device of the present invention will be described with reference to the drawings. In the following description, a front-rear direction, a left-right direction, and an up-down direction are defined with reference to a user of the electric prosthetic leg. In the drawings, front, rear, left, right, upper, and lower sides of the electric prosthetic leg are represented by Fr, Rr, L, R, U, and D, respectively.

### [Electric Prosthetic Leg]

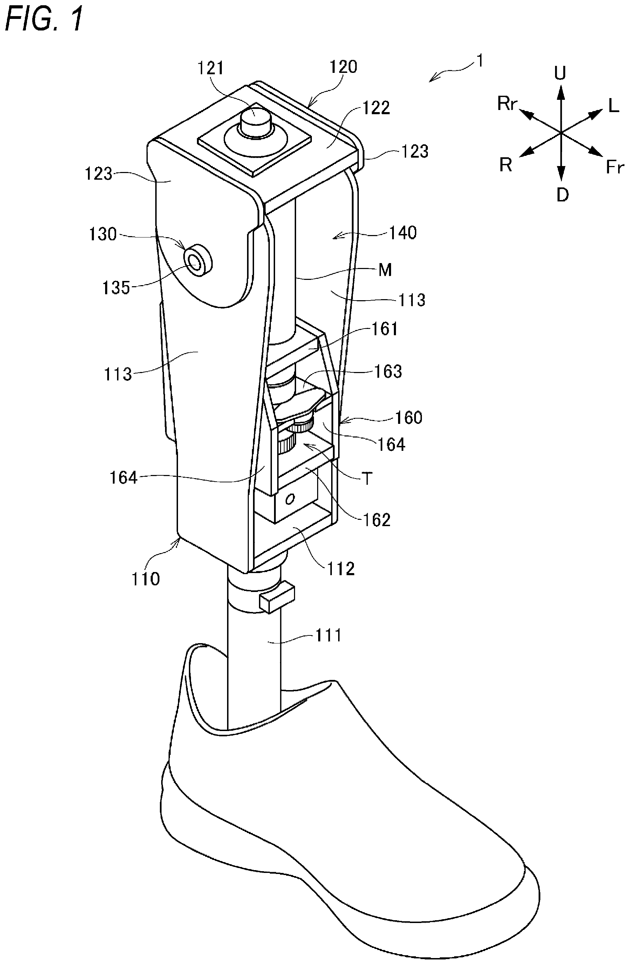

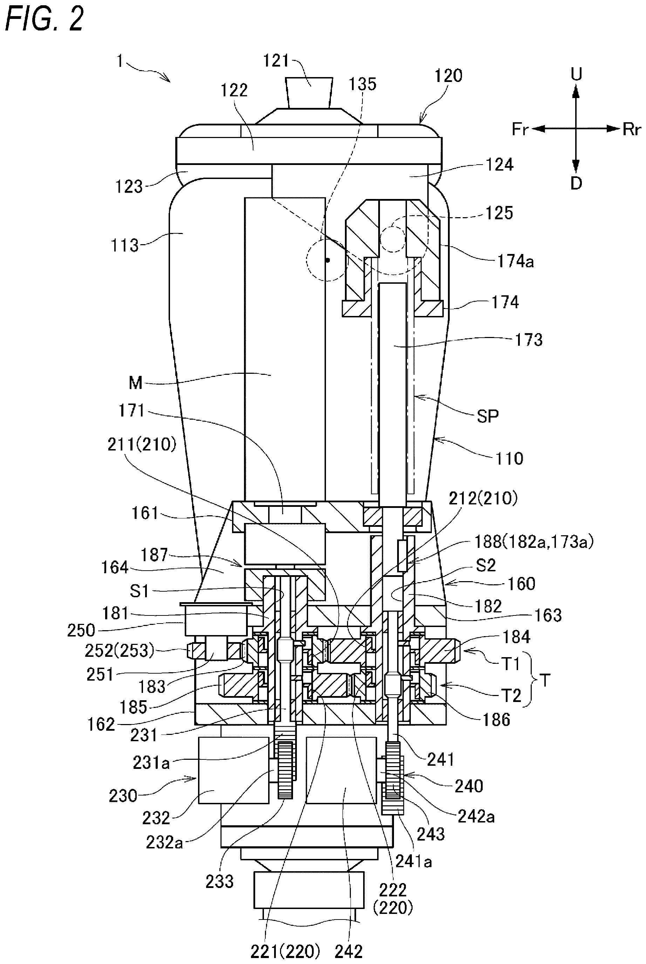

As shown in Figs. 1 and 2, an electric prosthetic leg 1 according to a first embodiment is a prosthetic leg that is attached to a leg portion of a person who does not have a knee, and includes a knee lower side member 110 that is positioned at a lower side of the knee, a knee upper side member 120 that is attached to a thigh and positioned above the knee, a knee joint mechanism 130 that connects the knee lower side member 110 and the knee upper side member 120 such that an angle formed by the knee lower side member 110 and the knee upper side member 120 is changeable, an expansion and contraction device 140 that is configured to change the angle formed by the knee lower side member 110 and the knee upper side member 120 by expansion and contraction, and a battery (not shown).

The knee upper side member 120 includes an upper wall portion 122 provided with an adapter 121 connected to a socket (not shown), and a pair of upper side wall portions 123 extending downward from both left and right ends of the upper wall portion 122, and has a substantially U shape that opens downward when viewed from the front-rear direction.

The knee lower side member 110 includes a lower wall portion 112 provided with a leg portion 111, and a pair of lower side wall portions 113 extending upward from both left and right ends of the lower wall portion 112, and has a substantially U shape that opens upward when viewed from the front-rear direction.

The pair of lower side wall portions 113 of the knee lower side member 110 are connected between the pair of upper side wall portions 123 of the knee upper side member 120 so as to be pivotable about pivot portions 135. With this mechanism, the knee joint mechanism 130 is formed by connecting the knee lower side member 110 and the knee upper side member 120 such that the angle formed by the knee lower side member 110 and the knee upper side member 120 is changeable.

In a space formed between the knee upper side member 120 and the knee lower side member 110, the expansion and contraction device 140 capable of changing the angle formed by the knee lower side member 110 and the knee upper side member 120 is provided.

The expansion and contraction device 140 includes a motor M that outputs a rotary power, a transmission T that transmits the power of the motor M, a spindle unit SP that is connected to the transmission T so as to transmit the power and converts the rotary power output from the transmission T into a translational motion, a first connection and disconnection mechanism 210 and a second connection and disconnection mechanism 220 provided on the transmission T, a first operation mechanism 230 and a second operation mechanism 240 that switch between the first connection and disconnection mechanism 210 and the second connection and disconnection mechanism 220, and a rotary damper 250 that damps an external force input from the spindle unit SP.

The transmission T includes a transmission case 160 including a top plate portion 161, a bottom plate portion 162, a middle plate portion 163 that is disposed in parallel between the top plate portion 161 and the bottom plate portion 162, and a pair of side plate portions 164 that connect left and right ends of the top plate portion 161, the bottom plate portion 162, and the middle plate portion 163, and having a rectangular shape when viewed in the front-rear direction. The transmission case 160 is swingably and immovably supported by the knee lower side member 110 via a lower swinging portion (not shown).

The motor M is disposed in front of and above the top plate portion 161 of the transmission case 160 such that an output shaft 171 passes through the top plate portion 161 and protrudes into the transmission case 160. The spindle unit SP is disposed on a side opposite to the motor M in the front-rear direction. The spindle unit SP includes a spindle 173 formed with a male screw and a sleeve 174 formed with a female screw, and the sleeve 174 performs the translational motion along a central axis of the spindle 173 by rotation of the spindle 173.

In the present embodiment, the spindle 173 receives the rotary power of the motor M transmitted by the transmission T to perform a rotational motion. Meanwhile, in the sleeve 174, a base portion 174a of the sleeve 174 is attached to a pair of inner side wall portions 124 extending downward from the upper wall portion 122 of the knee upper side member 120 so as to be swingable and immovable about an upper swing portion 125. Therefore, when the spindle 173 receives the rotary power of the motor M transmitted by the transmission T and rotates to one side, the sleeve 174 moves in a translational manner so as to separate from the transmission T, and when the spindle 173 rotates to the other side, the sleeve 174 moves in the translational manner so as to approach the transmission T. A motion that the sleeve 174 moves in the translational manner so as to separate from the transmission T is referred to as an extension motion of the spindle unit SP, and conversely, a motion that the sleeve 174 moves in the translational manner so as to approach the transmission T is referred to as a contraction motion of the spindle unit SP.

That is, a distance between the sleeve 174 and the transmission T increases or decreases in accordance with a rotation direction of the spindle 173. Since the sleeve 174 is immovably attached to the knee upper side member 120 as described above, the knee lower side member 110 to which the transmission T is attached and the knee upper side member 120 to which the sleeve 174 is attached pivot about the pivot portions 135 as the distance between the sleeve 174 and the transmission T increasing or decreasing in accordance with the rotation direction of the spindle 173. Accordingly, the angle formed by the knee upper side member 120 and the knee lower side member 110 changes. Assuming that the angle formed by the knee upper side member 120 and the knee lower side member 110 is an acute angle between the acute angle and an obtuse angle, the knee joint mechanism 130 extends when the formed angle increases, and the knee joint mechanism 130 flexes when the formed angle decreases.

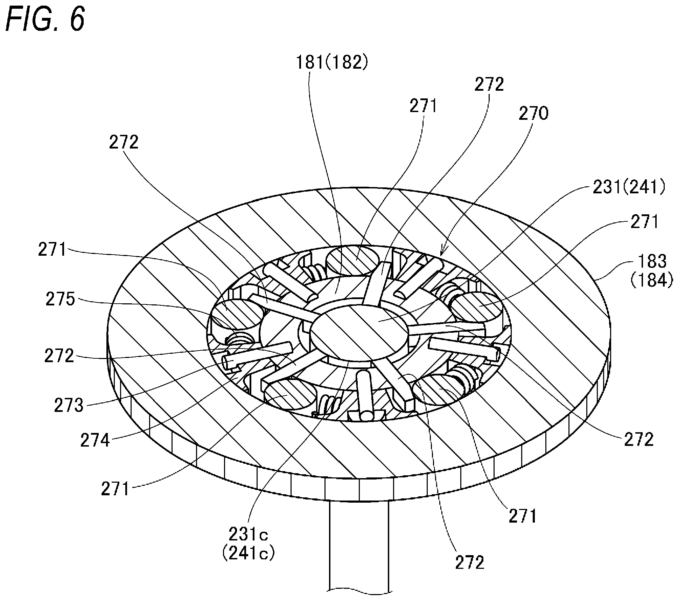

As shown in Figs. 2 to 6, the transmission T includes a first transmission mechanism T1 that transmits the power of the motor M to the spindle unit SP at a first transmission gear ratio, and a second transmission mechanism T2 that transmits the power of the motor M to the spindle unit SP at a second transmission gear ratio different from the first transmission gear ratio. The first transmission mechanism T1 is switched between a power interruption state and a power connection state by the first connection and disconnection mechanism 210, and the second transmission mechanism T2 is switched between the power interruption state and the power connection state by the second connection and disconnection mechanism 220.

According to such a transmission T, by providing two power transmission paths having different transmission gear ratios, motion speeds and generation powers of extension and flexion in the knee joint mechanism 130 can be switched. It is sufficient that the first transmission gear ratio and the second transmission gear ratio are different from each other, and either one of the first transmission mechanism T1 and the second transmission mechanism T2 may be a speed reducing mechanism and the other may be a speed increasing mechanism, either one may be a constant speed mechanism and the other may be the speed reducing mechanism or the speed increasing mechanism, both may be the speed reducing mechanisms, or both may be the speed increasing mechanisms.

The first transmission gear ratio is a ratio of a post-shift rotation speed, which is a rotation speed on a side opposite to a motor M side (spindle unit SP side) in the first transmission mechanism T1, to a pre-shift rotation speed, which is a rotation speed on the motor M side in the first transmission mechanism T1. The second transmission gear ratio is a ratio of the post-shift rotation speed, which is a rotation speed on the side opposite to the motor M side (spindle unit SP side) in the second transmission mechanism T2, to the pre-shift rotation speed, which is the rotation speed on the motor M side in the second transmission mechanism T2.

For example, when the first transmission gear ratio of the first transmission mechanism T1 is smaller than 1, the rotation speed on the side opposite to the motor M side (spindle unit SP side) is lower than the rotation speed on the motor M side, and a torque increases. When the second transmission gear ratio of the second transmission mechanism T2 is larger than 1, the rotation speed on the side opposite to the motor M side (spindle unit SP side) increases more than the rotation speed on the motor M side, and the torque decreases. In the present embodiment, the first transmission gear ratio is set to be smaller than 1, the second transmission gear ratio is set to be larger than 1, and a diameter of a first drive gear 183 is smaller than that of a second drive gear 185. In the present embodiment, the first transmission mechanism T1 is disposed above the second transmission mechanism T2.

The first transmission mechanism T1 and the second transmission mechanism T2 include a first shaft 181 rotatably disposed on a downward extension line of the output shaft 171 of the motor M, and a second shaft 182 rotatably disposed on a downward extension line of the spindle 173 of the spindle unit SP. The first shaft 181 is connected, via a coupling 187 that allows for an axial center error, to the output shaft 171 of the motor M so as to be integrally rotatable, and the second shaft 182 is connected, via a key 188 and key grooves 182a and 173a, to the spindle 173 of the spindle unit SP so as to be integrally rotatable. The output shaft 171 of the motor M and the first shaft 181 may be connected by key fitting or spline fitting without using the coupling 187. The spindle 173 and the second shaft 182 of the spindle unit SP may be connected using the spline fitting or coupling instead of the key fitting.

The first transmission mechanism T1 includes the first drive gear 183 and a first driven gear 184 that mesh with each other. The first drive gear 183 is rotatably supported by the first shaft 181 so as to allow relative rotation, and the first driven gear 184 is supported by the second shaft 182 so as to allow the relative rotation. The first transmission mechanism T1 of the present embodiment is a speed reduction transmission mechanism in which the diameter of the first drive gear 183 is smaller than that of the first driven gear 184, and can extend and contract the spindle unit SP at a low speed and a high torque.

The second transmission mechanism T2 includes the second drive gear 185 and a second driven gear 186 that mesh with each other. The second drive gear 185 is rotatably supported by the first shaft 181 so as to allow the relative rotation, and the second driven gear 186 is supported by the second shaft 182 so as to allow the relative rotation. The second transmission mechanism T2 of the present embodiment is a speed increase transmission mechanism in which the diameter of the second drive gear 185 is larger than that of the second driven gear 186, and can extend and contract the spindle unit SP at a high speed and a low torque.

The first connection and disconnection mechanism 210 includes a first connection and disconnection portion 211 provided between the first drive gear 183 and the first shaft 181, and a second connection and disconnection portion 212 provided between the first driven gear 184 and the second shaft 182.

The second connection and disconnection mechanism 220 includes a third connection and disconnection portion 221 provided between the second drive gear 185 and the first shaft 181, and a fourth connection and disconnection portion 222 provided between the second driven gear 186 and the second shaft 182.

These connection and disconnection portions 211, 212, 221, and 222 have a common configuration, and are configured to be switched between an interruption state in which power transmission is interrupted and a power transmissible state in which the rotary power can be transmitted in two directions of one direction and another direction.

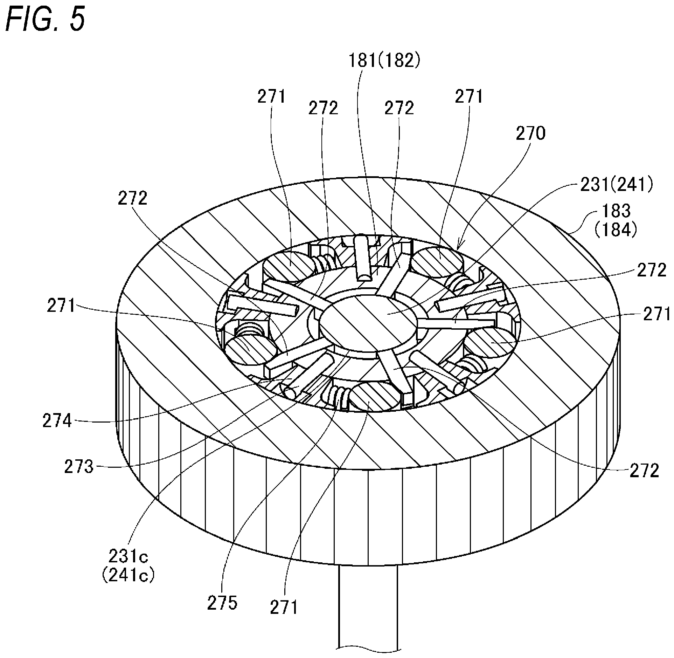

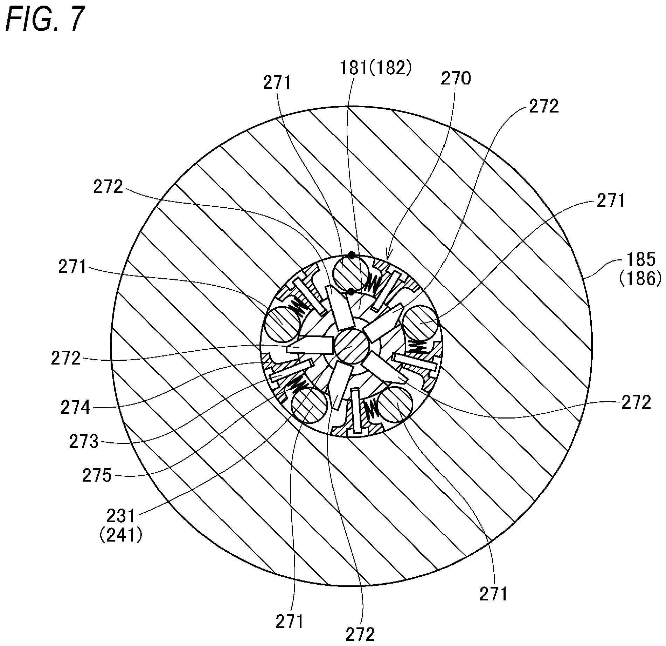

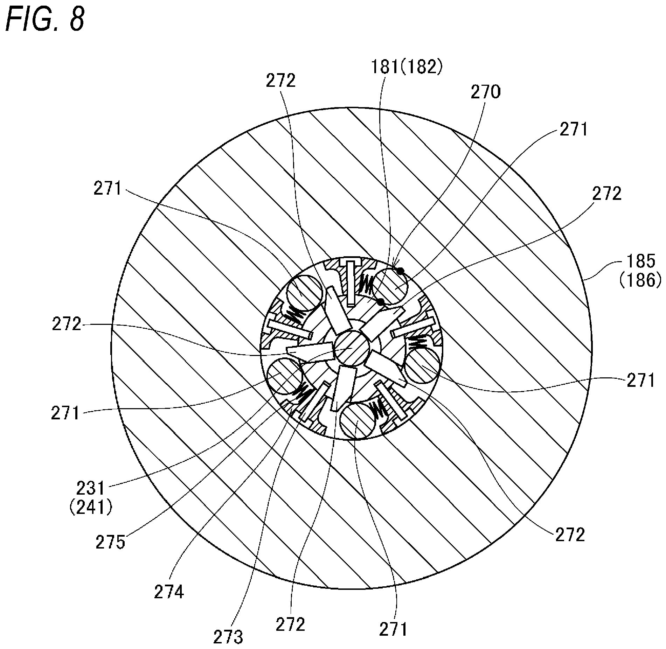

As shown in Figs. 5 to 8, each of the connection and disconnection portions 211, 212, 221, and 222 of the present embodiment is implemented by combining two one-way clutches 270 having a forced free function. Each of the one-way clutches 270 includes a plurality of rollers 271 that are disposed between each of outer peripheral surfaces of the shafts 181 and 182 and each of inner peripheral surfaces of the gears 183 to 186, are engaged when a rotary power in the one direction is input from a shaft side or a gear side and transmit the rotary power, and are disengaged when the rotary power in another direction is input from the shaft side or the gear side and interrupt the rotary power, a retainer 274 that holds the plurality of rollers 271 at predetermined intervals, and a plurality of pins 272 that forcefully hold the plurality of rollers 271 in a disengaged position to interrupt the rotary power in the one direction and another direction. In the drawings, a reference numeral 273 denotes a fixing pin that fixes the retainer 274 to each of the shafts 181 and 182, and a reference numeral 275 denotes a spring that biases the roller 271 from a retainer 274 side toward a pin 272 side. Each of the connection and disconnection portions 211, 212, 221, and 222 is implemented by stacking the two one-way clutches 270 such that a direction of rotation to be transmitted is reversed. Such connection and disconnection portions 211, 212, 221, and 222 are switched between the interruption state in which the power transmission is interrupted by forcing the two one-way clutches 270 to be free and the power transmissible state in which either one of the two one-way clutches 270 can be engaged to transmit the rotary power in the one direction and another direction.

The first operation mechanism 230 includes a first operation rod 231 provided so as to be able to operate the pins 272 of the first connection and disconnection portion 211 of the first connection and disconnection mechanism 210 and the pins 272 of the third connection and disconnection portion 221 of the second connection and disconnection mechanism 220, and a first servomotor 232 that causes the first operation rod 231 to linearly move. An operation rod provided to be able to operate the pins 272 of the first connection and disconnection portion 211 and an operation rod provided to be able to operate the pins 272 of the third connection and disconnection portion 221 are different, and a servomotor may be provided to cause each operation rod to linearly move.

The second operation mechanism 240 includes a second operation rod 241 provided so as to be able to operate the pins 272 of the second connection and disconnection portion 212 of the first connection and disconnection mechanism 210 and the pins 272 of the fourth connection and disconnection portion 221 of the second connection and disconnection mechanism 220, and a second servomotor 242 that causes the second operation rod 241 to linearly move. An operation rod provided to be able to operate the pins 272 of the second connection and disconnection portion 212 and an operation rod provided to be able to operate the pins 272 of the fourth connection and disconnection portion 222 are different, and a servomotor may be provided to cause each operation rod to linearly move.

The first shaft 181 is a hollow shaft having a first internal space S1 extending in a rotation axis direction, and the second shaft 182 is a hollow shaft having a second internal space S2 extending in the rotation axis direction. The first operation rod 231 is disposed so as to be movable in the up-down direction in the first internal space S1, and the second operation rod 241 is disposed so as to be movable in the up-down direction in the second internal space S2. The first shaft 181 and the second shaft 182 are disposed so as to extend in a vertical direction when a user of the electric prosthetic leg 1 stands upright.

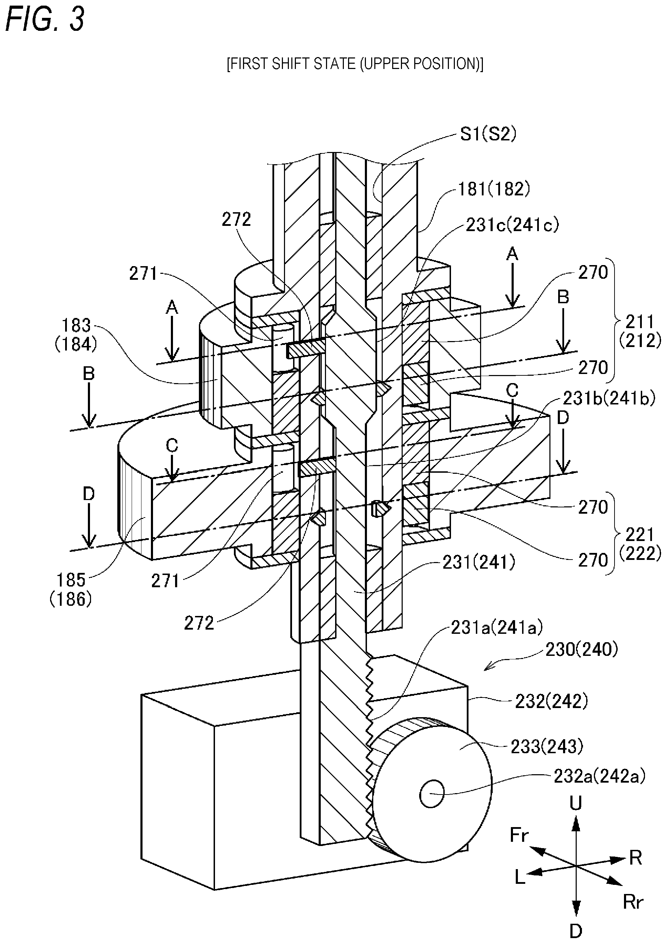

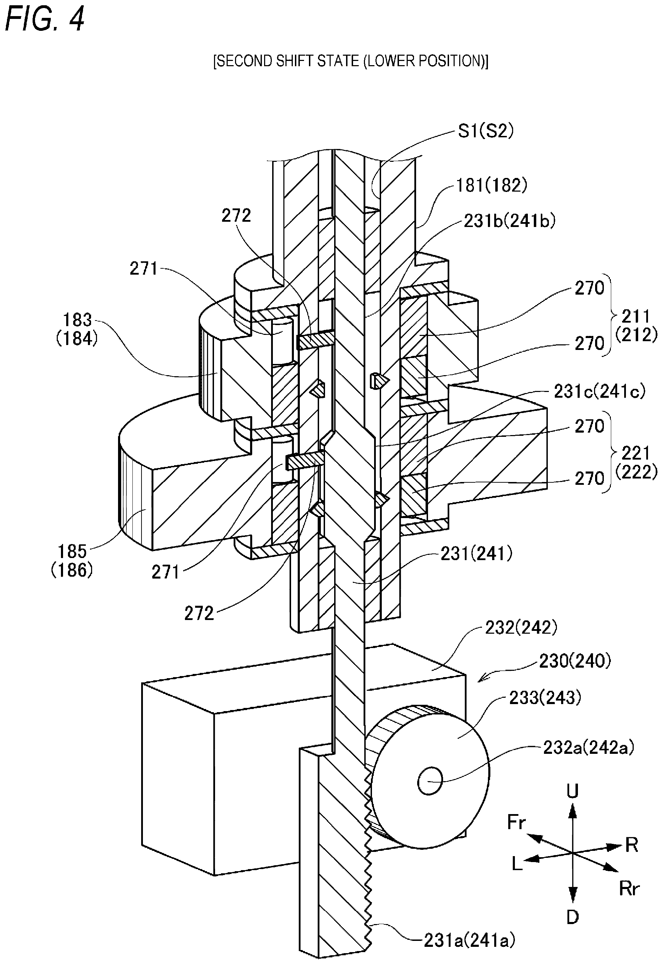

The first operation rod 231 has a rack 231a on a lower end side. A pinion 233 provided on an output shaft 232a of the first servomotor 232 meshes with the rack 231a, and a position of the first operation rod 231 is switched between an upper position shown in Fig. 3 and a lower position shown in Fig. 4 according to drive of the first servomotor 232. Figs. 3 to 6 show the first operation mechanism 230, and the second operation mechanism 240 has the same configuration. Reference numerals in parentheses in Figs. 3 to 8 denote components of the second operation mechanism 240 corresponding to components of the first operation mechanism 230.

The second operation rod 241 has a rack 241a on a lower end side. A pinion 243 provided on an output shaft 242a of the second servomotor 242 meshes with the rack 241a, and a position of the second operation rod 241 is switched between an upper position and a lower position according to drive of the second servomotor 242.

The pins 272 of the connection and disconnection portion 211, 212, 221, and 222 are provided movably in a diameter direction with respect to rotation axes of the first shaft 181 and the second shaft 182, and the first operation rod 231 and the second operation rod 241 are provided such that outer peripheral portions come into contact with inner end portions of the pins 272. The outer peripheral portion of the first operation rod 231 and the outer peripheral portion of the second operation rod 241 respectively include small diameter portions 231b and 241b that cause the pins 272 to be positioned inward in a forced free release position, and large diameter portions 231c and 241c that push the pins 272 outward to a forced free position. Between the small diameter portions 231b and 241b and the large diameter portions 231c and 241c, inclination portions are provided to connect the small diameter portions 231b and 241b and the large diameter portions 231c and 241c without steps.

The present embodiment has a first shift state in which the first operation rod 231 and the second operation rod 241 are positioned at the upper position, and a second shift state in which the first operation rod 231 and the second operation rod 241 are positioned at the lower position. In the first shift state, as shown in Figs. 3 and 5 to 8, the large diameter portions 231c and 241c of the first operation rod 231 and the second operation rod 241 force the first connection and disconnection portion 211 and the second connection and disconnection portion 212 of the first connection and disconnection mechanism 210 to be free, so that the motor M and the spindle unit SP enter a power transmission state via the second transmission mechanism T2. In the second shift state, as shown in Fig. 4, the large diameter portions 231c and 241c of the first operation rod 231 and the second operation rod 241 force the third connection and disconnection portion 221 and the fourth connection and disconnection portion 222 of the second connection and disconnection mechanism 220 to be free, so that the motor M and the spindle unit SP enter the power transmission state via the first transmission mechanism T1.

An external force in a flexion direction input from the spindle unit SP is transmitted to the rotary damper 250 via the first transmission mechanism T1. Specifically, an input shaft 251 of the rotary damper 250 is provided with an input gear 252 that meshes with the first drive gear 183 of the first transmission mechanism T1. A one-way clutch 253 is provided between the input shaft 251 and the input gear 252 to transmit rotation of the first transmission mechanism T1 in the one direction to the rotary damper 250 and to interrupt the rotation in an opposite direction. Accordingly, even in the first shift state, when the motor M is power-driven, power transmission to the rotary damper 250 is interrupted, and when the motor M is not power-driven (is subjected to zero torque control or regeneration control), an external force input from the spindle unit SP can be transmitted to the rotary damper 250 and damped.

The electric prosthetic leg 1 configured as described above can smoothly perform a going-up-stairs motion that needs to be performed by a passive prosthetic leg including a passive damper in the related art with a leg on a non-prosthetic side going up one stair per time.

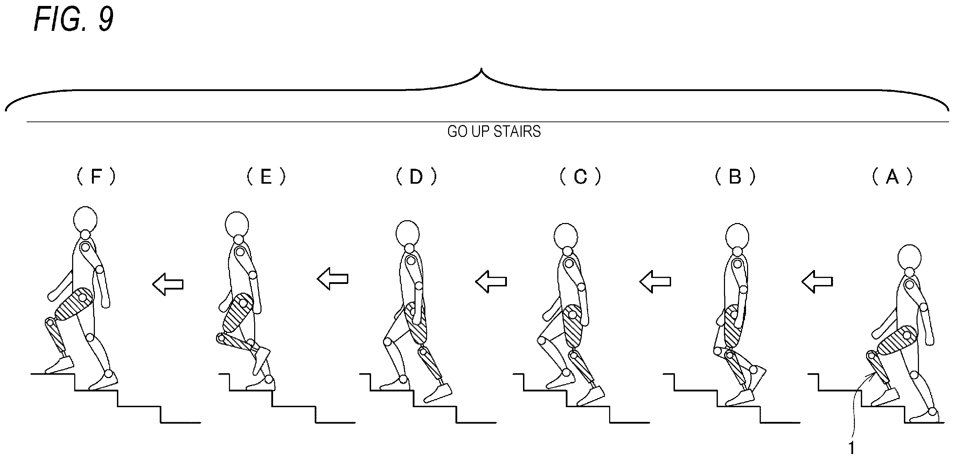

Specifically, as shown in part (A) → part (B) of Fig. 9, in a state in which a load is applied to the electric prosthetic leg 1 when the electric prosthetic leg 1 is pushed forward to go up the stairs, a large power is necessary when the knee joint mechanism 130 is extended from a flexed state.

In this case, the transmission T is set to the second shift state in which the first operation rod 231 and the second operation rod 241 are positioned at the lower position. In the second shift state, the large diameter portions 231c and 241c of the first operation rod 231 and the second operation rod 241 force the third connection and disconnection portion 221 and the fourth connection and disconnection portion 222 of the second connection and disconnection mechanism 220 to be free, so that the motor M and the spindle unit SP enter the power transmission state via the first transmission mechanism T1.

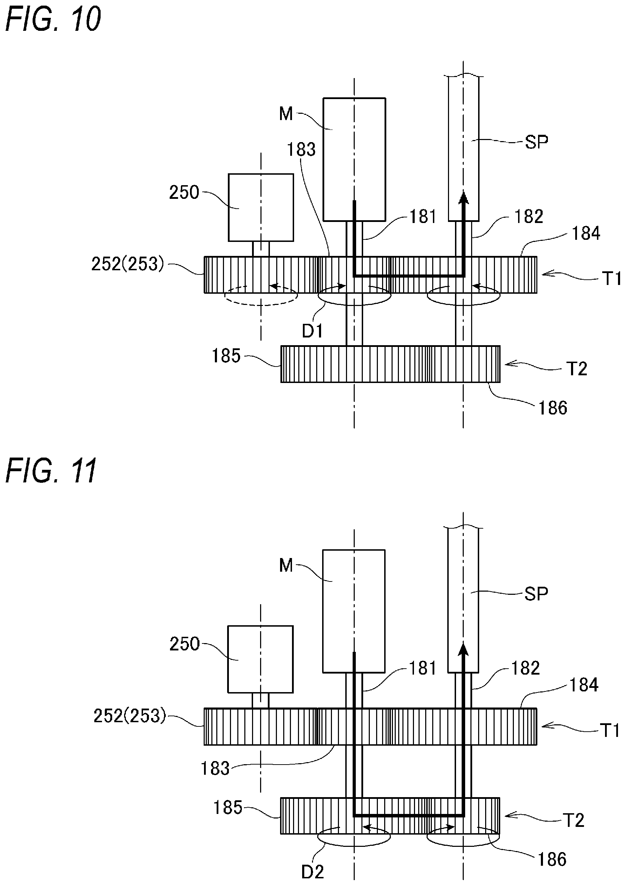

In this state, when the motor M is rotated in a first direction (D1 direction in Fig. 10), the power of the motor M is transmitted to the first shaft 181, the first connection and disconnection portion 211 of the first connection and disconnection mechanism 210, the first drive gear 183, the first driven gear 184, the second connection and disconnection portion 212 of the first connection and disconnection mechanism 210, the second shaft 182, and the spindle unit SP. Accordingly, the sleeve 174 moves in the translational manner (extends) so as to move away from the transmission T, and the knee upper side member 120 to which the sleeve 174 is attached is pivotable about the pivot portions 135 with respect to the knee lower side member 110 to which the transmission T is attached, and thereby the knee joint mechanism 130 extends. Since the power for extension is a power with high torque in a case of being decelerated by the first transmission mechanism T1, the knee joint mechanism 130 can be reliably extended from the flexed state even in a state in which a large load is applied to the electric prosthetic leg 1 when the electric prosthetic leg 1 is pushed forward to go up the stairs.

Meanwhile, in order to smoothly perform the going-up-stairs motion, as shown in part (D) → part (E) of Fig. 9, in a state in which a load is applied to a healthy leg, it is necessary to flex (lift) the knee joint mechanism 130 from an extension state. When the knee joint mechanism 130 is flexed from the extension state, a large power is not required, but a quick motion is required.

In this case, the transmission T is set to the first shift state in which the first operation rod 231 and the second operation rod 241 are positioned at the upper position. In the first shift state, the large diameter portions 231c and 241c of the first operation rod 231 and the second operation rod 241 force the first connection and disconnection portion 211 and the second connection and disconnection portion 212 of the first connection and disconnection mechanism 210 to be free, so that the motor M and the spindle unit SP enter the power transmission state via the second transmission mechanism T2.

In this state, when the motor M is rotated in a second direction (D2 direction in Fig. 11), the power of the motor M is transmitted to the first shaft 181, the third connection and disconnection portion 221 of the second connection and disconnection mechanism 220, the second drive gear 185, the second driven gear 186, the fourth connection and disconnection portion 222 of the second connection and disconnection mechanism 220, the second shaft 182, and the spindle unit SP. Accordingly, the sleeve 174 moves in the translational manner (contracts) so as to move close to the transmission T, and the knee lower side member 110 to which the transmission T is attached is pivotable about the pivot portions 135 with respect to the knee upper side member 120 to which the sleeve 174 is attached, and thereby the knee joint mechanism 130 is flexed. Since the power for flexing is a power with low torque in a case of being accelerated by the second transmission mechanism T2, the knee joint mechanism 130 can be flexed quickly.

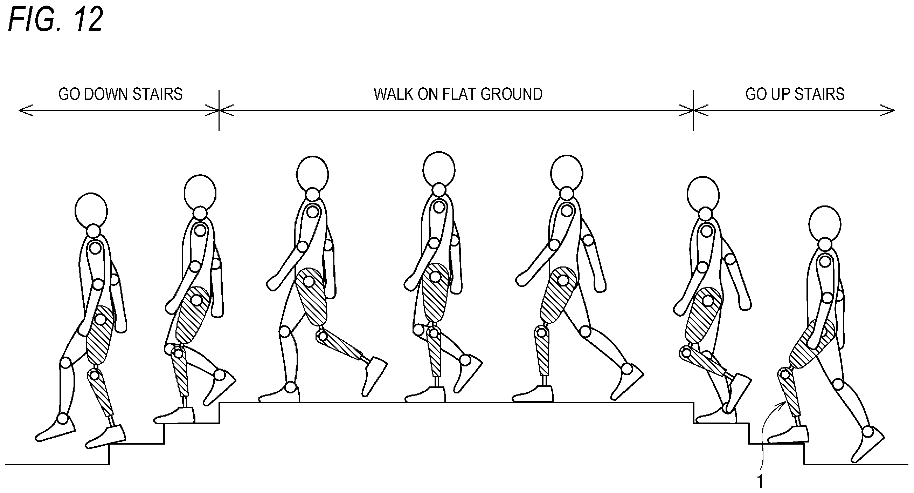

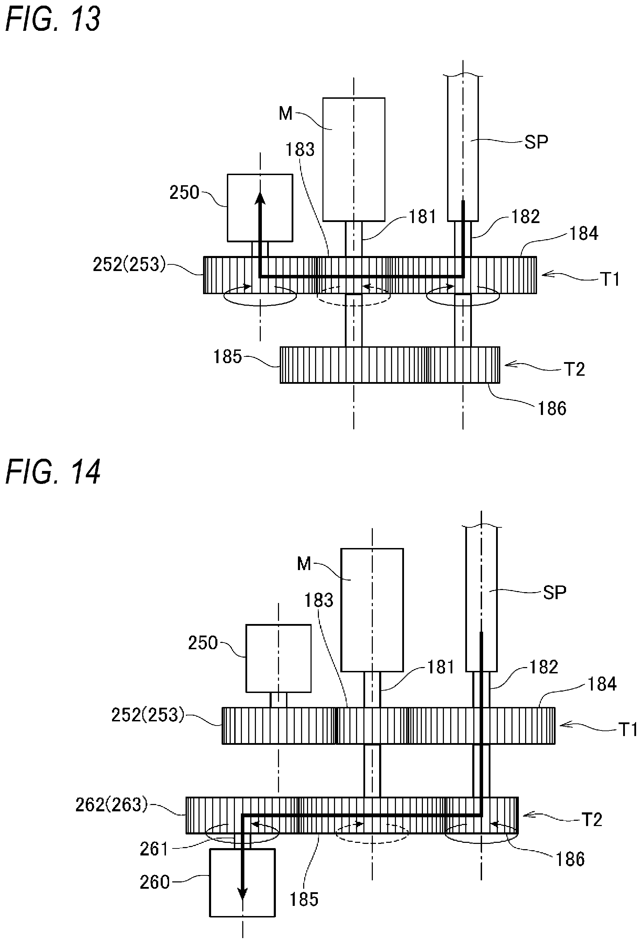

In cases of going down the stairs shown in Fig. 12 and walking on a flat ground, as shown in Fig. 13, by damping the external force in the flexion direction input from the spindle unit SP with the rotary damper 250, the knee joint mechanism 130 can be smoothly flexed.

In this case, the transmission T is set to the second shift state in which the first operation rod 231 and the second operation rod 241 are positioned at the lower position. In the second shift state, the large diameter portions 231c and 241c of the first operation rod 231 and the second operation rod 241 force the third connection and disconnection portion 221 and the fourth connection and disconnection portion 222 of the second connection and disconnection mechanism 220 to be free, so that the motor M and the spindle unit SP enter the power transmission state via the first transmission mechanism T1.

In this state, when the motor M is subjected to the zero torque control, the external force in the flexion direction input from the spindle unit SP is transmitted to the second shaft 182, the second connection and disconnection portion 212 of the first connection and disconnection mechanism 210, the first driven gear 184, the first drive gear 183, the input gear 252, the one-way clutch 253, and the rotary damper 250. Accordingly, the external force in the flexion direction input from the spindle unit SP is damped by the rotary damper 250, and the knee joint mechanism 130 can be smoothly flexed. The motor M may be subjected to the regeneration control instead of the zero torque control. Thus, damping performance at the time of flexing can be enhanced.

Next, a modification of the electric prosthetic leg 1 of the first embodiment will be described with reference to Fig. 14. However, the same reference numerals as those of the embodiment are used for configurations common to those of the embodiment, and the description of the embodiment may be incorporated.

As shown in Fig. 14, the electric prosthetic leg 1 according to the modification differs from the embodiment in that the electric prosthetic leg of the modification includes a second rotary damper 260 that damps an external force in an extension direction input from the spindle unit SP during walking on the flat ground.

The external force in the extension direction input from the spindle unit SP is transmitted to the second rotary damper 260 via the second transmission mechanism T2. Specifically, an input shaft 261 of the second rotary damper 260 is provided with an input gear 262 that meshes with the second drive gear 185 of the second transmission mechanism T2. A one-way clutch 263 is provided between the input shaft 261 and the input gear 262 to transmit rotation of the second transmission mechanism T2 in the one direction to the second rotary damper 260 and interrupt rotation in an opposite direction. Accordingly, even in the first shift state, when the motor M is power-driven, power transmission to the second rotary damper 260 is interrupted, and when the motor M is not power-driven (is subjected to the zero torque control or the regeneration control), the external force in the extension direction input from the spindle unit SP can be transmitted to the second rotary damper 260 and damped.

Specifically, when the external force in the extension direction input from the spindle unit SP is damped by the second rotary damper 260, the transmission T is set to the first shift state in which the first operation rod 231 and the second operation rod 241 are positioned at the upper position. In the first shift state, the large diameter portions 231c and 241c of the first operation rod 231 and the second operation rod 241 force the first connection and disconnection portion 211 and the second connection and disconnection portion 212 of the first connection and disconnection mechanism 210 to be free, so that the motor M and the spindle unit SP enter the power transmission state via the second transmission mechanism T2.

In this state, when the motor M is subjected to the zero torque control, the external force in the extension direction input from the spindle unit SP is transmitted to the second shaft 182, the fourth connection and disconnection portion 222 of the second connection and disconnection mechanism 220, the second driven gear 186, the second drive gear 185, the input gear 262, the one-way clutch 263, and the second rotary damper 260. Accordingly, the external force in the extension direction input from the spindle unit SP is damped by the second rotary damper 260, and the knee joint mechanism 130 can smoothly extend. The motor M may be subjected to the regeneration control instead of the zero torque control. Thus, the damping performance at the time of extending can be enhanced.

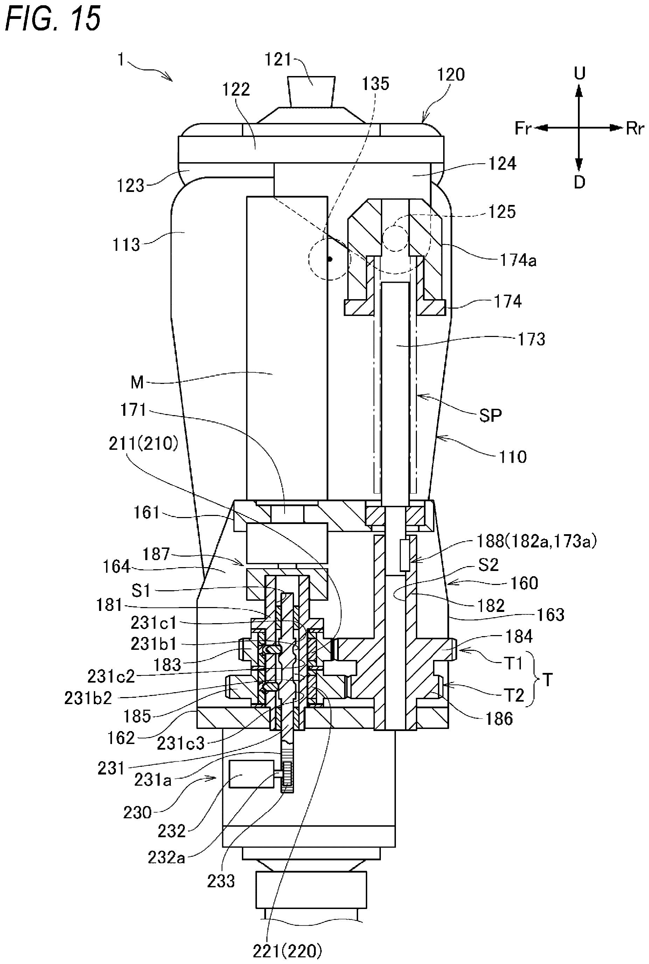

Next, the electric prosthetic legs 1 according to a second embodiment and a third embodiment of the present invention will be described with reference to Figs. 15 to 26. However, descriptions of the same configurations as those in the first embodiment are omitted, or the same configurations are denoted by the same reference numerals as those in the first embodiment and the description of the first embodiment may be incorporated.

The transmission T according to the first embodiment described above includes four two-way clutches (connection and disconnection portions 211, 212, 221, and 222) each implemented by combining two one-way clutches 270 having the forced free function, and switches these two-way clutches on and off with two actuators (servomotors 232 and 242), whereas the transmissions T according to the second embodiment and the third embodiment are different in each including two two-way clutches having the forced free function, and switching these two-way clutches on and off by one actuator. According to such a second embodiment and such a third embodiment, the number of parts of the transmission T can be reduced, and the structure can be simplified and the cost can be reduced. Hereinafter, the configurations of the transmissions T according to the second and third embodiments and the configurations and operations of the two-way clutches according to the second and third embodiments will be sequentially described.

As shown in Fig. 15, similar to the transmission T according to the first embodiment, the transmission T according to the second embodiment includes the first transmission mechanism T1 that transmits the power of the motor M to the spindle unit SP at the first transmission gear ratio, and the second transmission mechanism T2 that transmits the power of the motor M to the spindle unit SP at the second transmission gear ratio different from the first transmission gear ratio. The first transmission mechanism T1 is switched between the power interruption state and the power connection state by the first connection and disconnection mechanism 210, and the second transmission mechanism T2 is switched between the power interruption state and the power connection state by the second connection and disconnection mechanism 220.

The first transmission mechanism T1 according to the second embodiment includes the first shaft 181 that is mechanically connected to the output shaft 171 of the motor M, the second shaft 182 that is mechanically connected to the spindle 173 of the spindle unit SP, the first drive gear 183 that is rotatably provided on the first shaft 181, and the first driven gear 184 that is provided on the second shaft 182 so as to be integrally rotatable and rotates synchronously with the first drive gear 183.

The second transmission mechanism T2 according to the second embodiment includes the first shaft 181, the second shaft 182, the second drive gear 185 that is provided on the first shaft 181 so as to allow the relative rotation, and the second driven gear 186 that is provided on the second shaft 182 so as to be integrally rotatable and rotates synchronously with the second drive gear 185.

The first connection and disconnection mechanism 210 according to the second embodiment includes the first connection and disconnection portion 211 that is provided between the first drive gear 183 and the first shaft 181, and the second connection and disconnection mechanism 220 includes the third connection and disconnection portion 221 that is provided between the second drive gear 185 and the first shaft 181. That is, in the transmission T according to the second embodiment, the connection and disconnection portion 211 is provided between the first shaft 181 and the gear 183, the connection and disconnection portion 221 is provided between the first shaft 181 and the gear 185, the connection and disconnection portion 212 is not provided between the second shaft 182 and the gear 184, and the connection and disconnection portion 222 is not provided between the second shaft 182 and the gear 186.

These connection and disconnection portions 211 and 221 have a common configuration, and are configured to be switched between the interruption state in which the power transmission is interrupted and the power transmissible state in which the rotary power can be transmitted in two directions of one direction and another direction. Details will be described later.

As shown in Fig. 16, similar to the transmission T according to the second embodiment, the transmission T according to the third embodiment includes the first transmission mechanism T1, the second transmission mechanism T, the first connection and disconnection mechanism 210, and the second connection and disconnection mechanism 220.

The first transmission mechanism T1 according to the third embodiment includes the first shaft 181, the second shaft 182, the first drive gear 183 that is provided on the first shaft 181 so as to be integrally rotatable, and the first driven gear 184 that is provided on the second shaft 182 so as to allow the relative rotation and rotates synchronously with the first drive gear 183.

The second transmission mechanism T2 according to the third embodiment includes the first shaft 181, the second shaft 182, the second drive gear 185 that is provided on the first shaft 181 so as to be integrally rotatable, and the second driven gear 186 that is provided on the second shaft 182 so as to allow the relative rotation and rotates synchronously with the second drive gear 185.

The first connection and disconnection mechanism 210 according to the third embodiment includes the second connection and disconnection portion 212 that is provided between the first driven gear 184 and the second shaft 182, and the second connection and disconnection mechanism 220 includes the fourth connection and disconnection portion 222 that is provided between the second driven gear 186 and the second shaft 182. That is, in the transmission T according to the third embodiment, the connection and disconnection portion 212 is provided between the second shaft 182 and the gear 184, the connection and disconnection portion 222 is provided between the second shaft 182 and the gear 186, the connection and disconnection portion 211 is not provided between the first shaft 181 and the gear 183, and the connection and disconnection portion 221 is not provided between the first shaft 181 and the gear 185.

These connection and disconnection portions 212 and 222 have a common configuration, and are configured to be switched between the interruption state in which the power transmission is interrupted and the power transmissible state in which the rotary power can be transmitted in the two directions of the one direction and another direction.

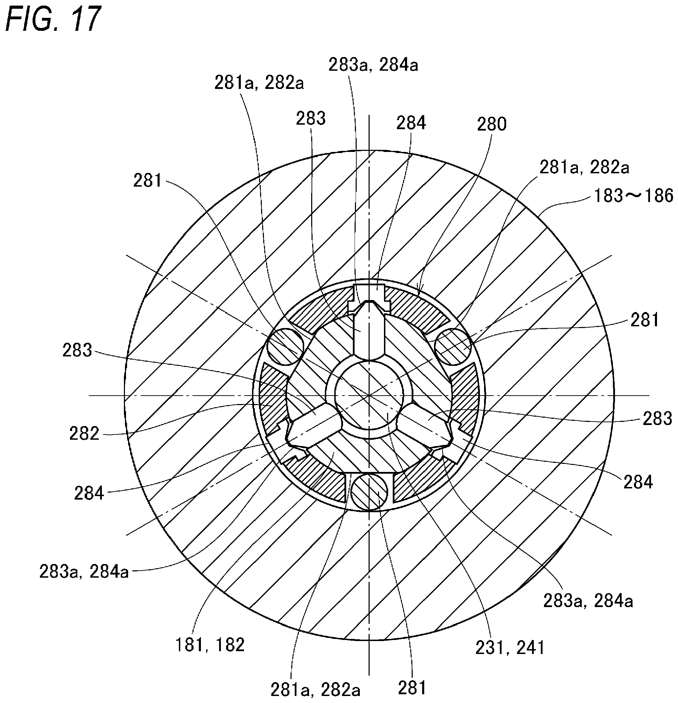

As shown in Fig. 17, each of the connection and disconnection portions 211 and 212 according to the second embodiment and the connection and disconnection portions 221 and 222 according to the third embodiment is implemented by combining two two-way clutches 280 having a forced free function. The two-way clutch 280 includes a plurality of (three in the present embodiment) rollers 281 disposed between each of the outer peripheral surfaces of the shafts 181 and 182 and each of the inner peripheral surfaces of the gears 183 to 186, a retainer 282 that holds the plurality of rollers 281 at predetermined intervals, a plurality of (three in the present embodiment) pins 283 that radially pass through each of the shafts 181 and 182 and are operated to the forced free position and the forced free release position by the first operation mechanism 230 or the second operation mechanism 240, and a plurality (three in the present embodiment) of guides 284 that is provided on the retainer 282 and defines a relative rotation position of the retainer 282 with respect to each of the shafts 181 and 182 when the pin 283 is in the forced free position.

An interval A (not shown) in the diameter direction between each of the outer peripheral surfaces of the shafts 181 and 182 and each of the inner peripheral surfaces of the gears 183 to 186 is smaller than a diameter B (not shown) of the roller 281. Flat portions 281a and flat portions 282a are respectively formed on the outer peripheral portions of the shafts 181 and 182 at predetermined intervals in a circumferential direction, and the interval A is larger than the diameter B on a center side in the circumferential direction of each of the flat portions 281a and 282a.

That is, in a state in which the rollers 281 are held at the center of the flat portions 281a and 282a in the circumferential direction, the rollers 281 do not mesh with each of the outer peripheral surfaces of the shafts 181 and 182 and each of the inner peripheral surfaces of the gears 183 to 186, and the relative rotation between the shafts 181 and 182 and the gears 183 to 186 is allowed (forced free state).

Meanwhile, in a state in which the rollers 281 are allowed to move in the circumferential direction with respect to each of the shafts 181 and 182, the rollers 281 do not mesh with each of the outer peripheral surfaces of the shafts 181 and 182 and each of the inner peripheral surfaces of the gears 183 to 186, and the shafts 181 and 182 and the gears 183 to 186 are connected so as to be integrally rotatable in the two directions (forced free release state).

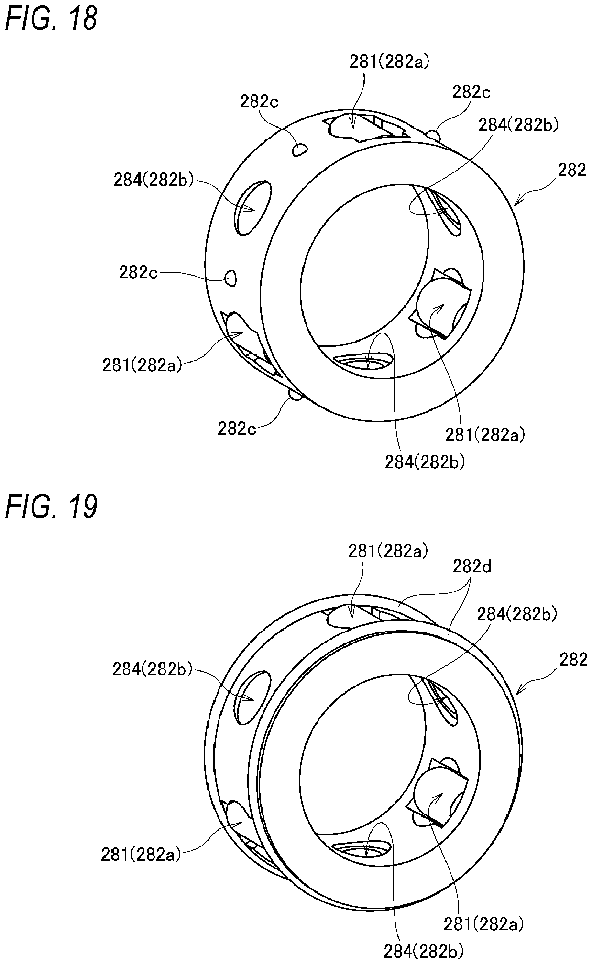

As shown in Fig. 18, the retainer 282 is ring-shaped and rotatable relative to each of the shafts 181 and 182 and each of the gears 183 to 186, and includes a plurality of roller holding portions 282a that hold the rollers 281 and a plurality of guide holding portions 282b that holds the guides 284.

A plurality of rubber bulbs 282c are embedded in an outer peripheral surface of the retainer 282 at predetermined intervals in the circumferential direction. These rubber bulbs 282c prevent unintended idling in the forced free release state by generating moderate friction between each of the gears 183 to 186 and the retainer 282. A member that generates friction between each of the gears 183 to 186 and the retainer 282 may include O-rings 282d as shown in Fig. 19. The rubber bulb 282c and the O-ring 282d are effective in preventing the idling, but can be omitted.

Returning to Fig. 17, the pin 283 has a conical convex portion 283a at an outer end in the diameter direction, and the guide 284 has a conical concave portion 284a that fits (engages) with the convex portion 283a on an inner end surface in the diameter direction. When the convex portion 283a of the pin 283 fits into the concave portion 284a of the guide 284, according to a guide action of the pin 283 and the guide 284, the relative rotation position of the retainer 282 with respect to each of the shafts 181 and 182 is positioned to a predetermined position which is in the forced free state.

As shown in Figs. 15 and 16, the shafts 181 and 182 are respectively formed with first large diameter portions 231c1 and 241c1, first small diameter portions 231b1 and 241b1, second large diameter portions 231c2 and 241c2, second small diameter portions 231b2 and 241b2, and third large diameter portions 231c3 and 241c3 with predetermined lengths and intervals in order from above. Each of the shafts 181 and 182 is provided so as to be able to simultaneously control the two connection and disconnection portions, but may be provided separately for each connection and disconnection portion.

In the following description, an operation of the second operation mechanism 240 that simultaneously controls the connection and disconnection portions 212 and 222 according to the third embodiment will be described with reference to Fig. 20.

As shown in Fig. 20, the connection and disconnection portions 212 and 222 are switched between the forced free state (hereinafter, appropriately referred to as an OFF state) and the forced free release state (hereinafter, appropriately referred to as an ON state) by the second operation mechanism 240.

When the second operation rod 241 of the second operation mechanism 240 is in an upper position shown in part (A) in Fig. 20, the second large diameter portion 241c2 pushes the pin 283 of the second connection and disconnection portion 212 radially outward, and the third large diameter portion 241c3 pushes the pin 283 of the fourth connection and disconnection portion 222 radially outward, so that the second connection and disconnection portion 212 and the fourth connection and disconnection portion 222 are in the OFF state.

When the second operation rod 241 of the second operation mechanism 240 is in a middle position shown in part (B) of Fig. 20, the first small diameter portion 241b1 allows the pin 283 of the second connection and disconnection portion 212 to return in an inner diameter direction, and the third large diameter portion 241c3 pushes the pin 283 of the fourth connection and disconnection portion 222 radially outward, so that the second connection and disconnection portion 212 is in the ON state and the fourth connection and disconnection portion 222 is in the OFF state.

When the second operation rod 241 of the second operation mechanism 240 is in a lower position shown in part (C) of Fig. 20, the first large diameter portion 241c1 pushes the pin 283 of the second connection and disconnection portion 212 radially outward, and the second small diameter portion 241b2 allows the pin 283 of the fourth connection and disconnection portion 222 to return in the inner diameter direction, so that the second connection and disconnection portion 212 is in the OFF state and the fourth connection and disconnection portion 222 is in the ON state.

Although not shown in the drawings, the connection and disconnection portions 211 and 221 according to the second embodiment are also switched between the forced free state and the forced free release state by the first operation mechanism 230. The first operation rod 231 of the first operation mechanism 230 is configured to move to an upper position (corresonding to the position in part (A) of Fig. 20), a middle position (corresonding to the position in part (B) of Fig. 20), and a lower position (corresonding to the position in part (C) of Fig. 20). When the first operation rod 231 of the first operation mechanism 230 is in the upper position, the pins 283 of the first connection and disconnection portion 211 and the third connection and disconnection portion 221 are pushed radially outward, so that the first connection and disconnection portion 211 and the third connection and disconnection portion 221 are in the OFF state, when the first operation rod 231 is in the middle position, the pin 283 of the first connection and disconnection portion 211 is allowed to return radially inward and the pin 283 of the third connection and disconnection portion 221 is pushed radially outward, so that the first connection and disconnection portion 211 is in the ON state, and the third connection and disconnection portion 221 is in the OFF state, and when the first operation rod 231 is in the lower position, the pin 283 of the first connection and disconnection portion 211 is pushed radially outward and the pin 283 of the third connection and disconnection portion 221 is allowed to return radially inward, so that the first connection and disconnection portion 211 is in the OFF state and the third connection and disconnection portion 221 is in the ON state.

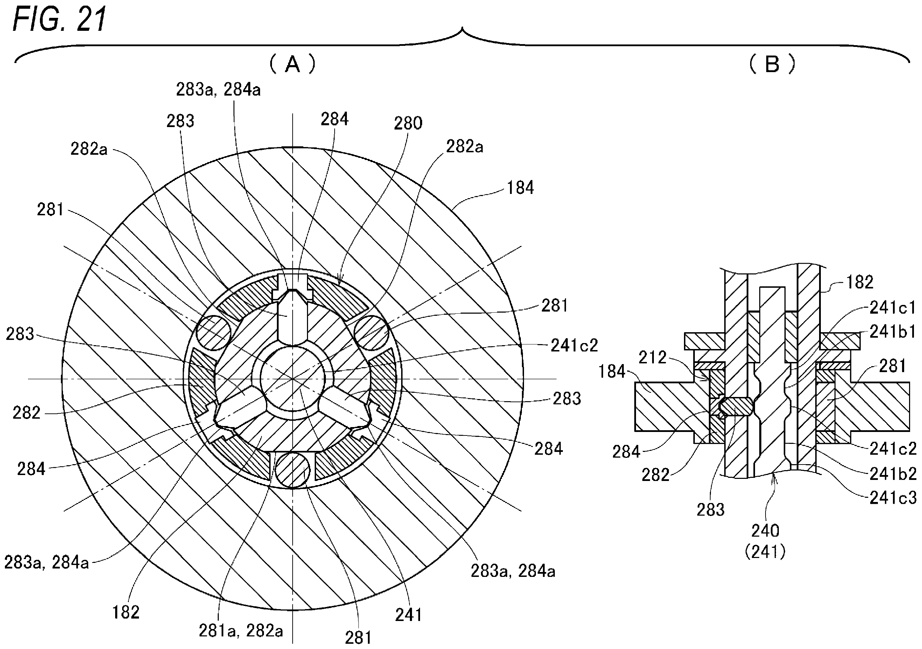

Next, the operation of the two-way clutch 280 will be described with reference to Figs. 21 to 26, taking the second connection and disconnection portion 212 according to the third embodiment as an example. In the following example, a case of transitioning from part (A) to part (C) via part (B) of Fig. 20 in the second connection and disconnection portion 212 will be described as an example.

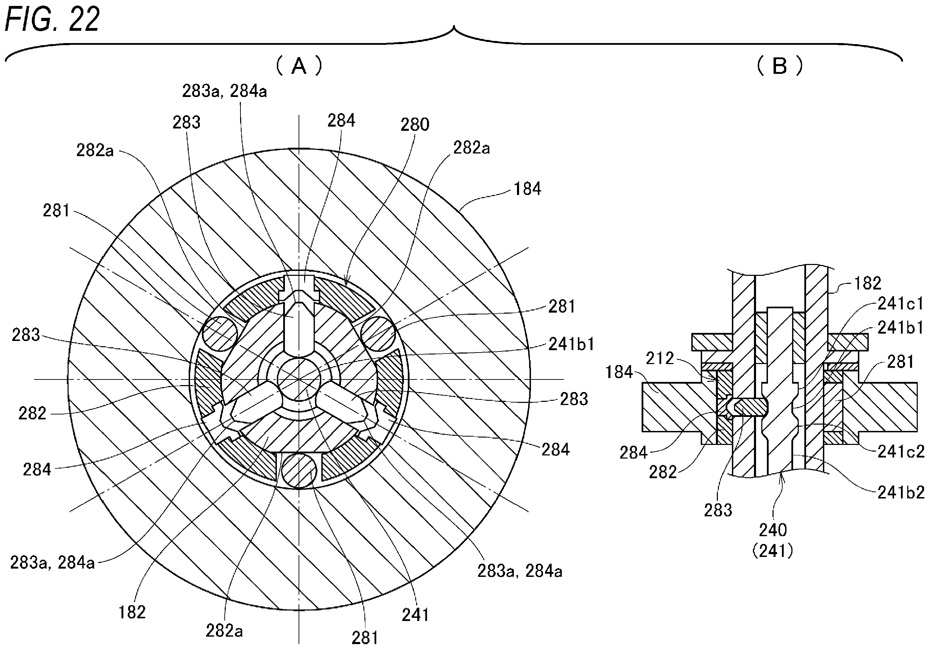

As shown in parts (A) and (B) of Fig. 21, in a state where the second large diameter portion 241c2 of the second operation rod 241 pushes the pin 283 of the second connection and disconnection portion 212 radially outward, the convex portion 283a of the pin 283 fits into the concave portion 284a of the guide 284, and the relative rotation position of the retainer 282 with respect to the second shaft 182 is fixed at a predetermined position. In this sate, the roller 281 is held at the center of the flat portion 282a in the circumferential direction, and thus the roller 281 does not mesh with the outer peripheral surface of the second shaft 182 and the inner peripheral surface of the first driven gear 184, and the OFF state is entered in which the relative rotation between the second shaft 182 and the first driven gear 184 is allowed.
parts (A) and (B) of Fig. 22 show a state in which the second operation rod 241 moves from a position where the second large diameter portion 241 c2 pushes the pin 283 of the second connection and disconnection portion 212 radially outward to a position where the first small diameter portion 241b1 allows the pin 283 to return radially inward. In Fig. 22, the pins 283 have already moved radially inward, and in fact, at a timing when the second shaft 182 and the first driven gear 184 rotate relative to each other, the guides 284 of the retainer 282 that rotates together with the first driven gear 184 push back the pins 283 in the diameter direction on inclined surfaces of the concave portions 284a.

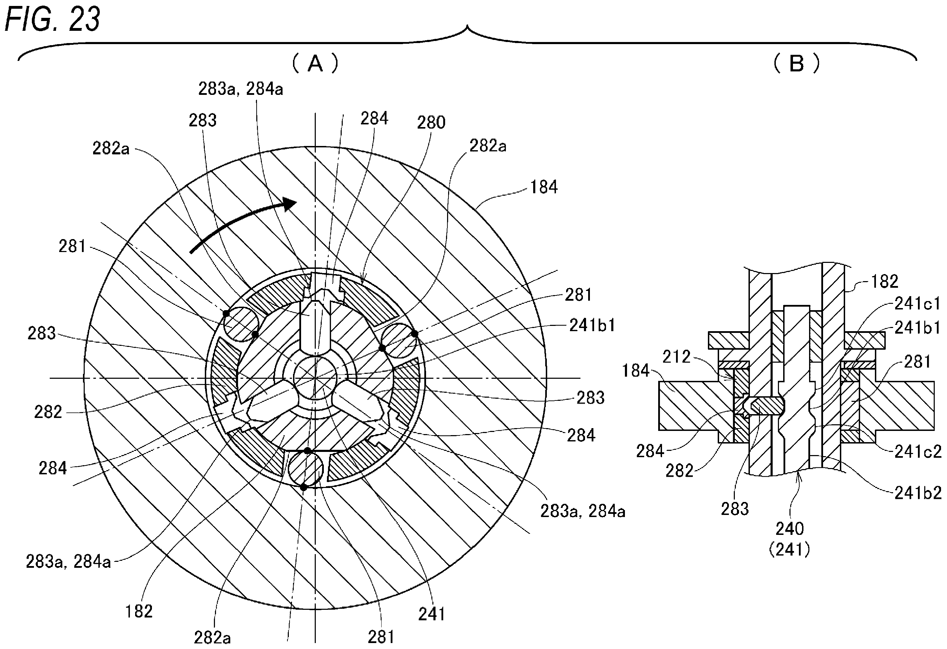

As shown in parts (A) and (B) of Fig. 23, in the state in which the pins 283 are allowed to return radially inward, when the second shaft 182 and the first driven gear 184 rotate relative to each other in a forward rotation direction indicated by an arrow in the drawing, the retainer 282 that rotates together with the first driven gear 184 causes the rollers 281 to move in the forward rotation direction with respect to the second shaft 182. Accordingly, the roller 281 meshes with the outer peripheral surface portion of the second shaft 182 and the inner peripheral surface portion of the first driven gear 184, and is in a forward rotation ON state in which the second shaft 182 and the first driven gear 184 are integrally rotated in the forward rotation direction.

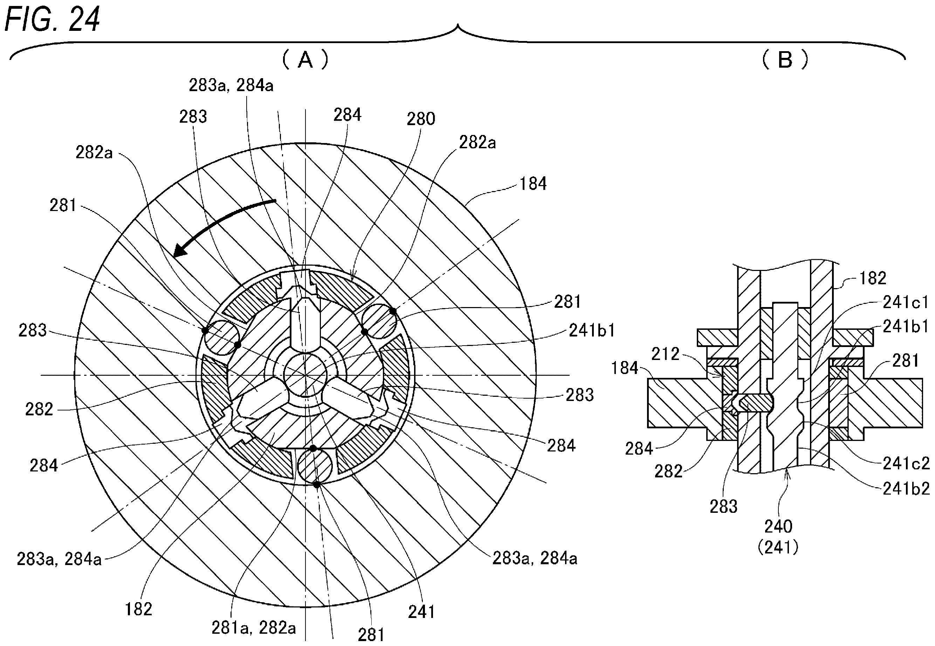

As shown in parts (A) and (B) of Fig. 24, in the state in which the pins 283 are allowed to return radially inward, when the second shaft 182 and the first driven gear 184 rotate relative to each other in a reverse rotation direction indicated by an arrow in the drawing, the retainer 282 that rotates together with the first driven gear 184 causes the rollers 281 to move in the reverse rotation direction with respect to the second shaft 182. Accordingly, the roller 281 meshes with the outer peripheral surface portion of the second shaft 182 and the inner peripheral surface portion of the first driven gear 184, and is in a reverse rotation OFF state in which the second shaft 182 and the first driven gear 184 are integrally rotated in the reverse rotation direction. The retainer 282 can be regarded as one element of the actuator of an operation unit that operates the roller 281, and can also be regarded as one element of an engager that is controlled by the pin 283 and the guide 284 to the forced free state position and the forced free release state position.

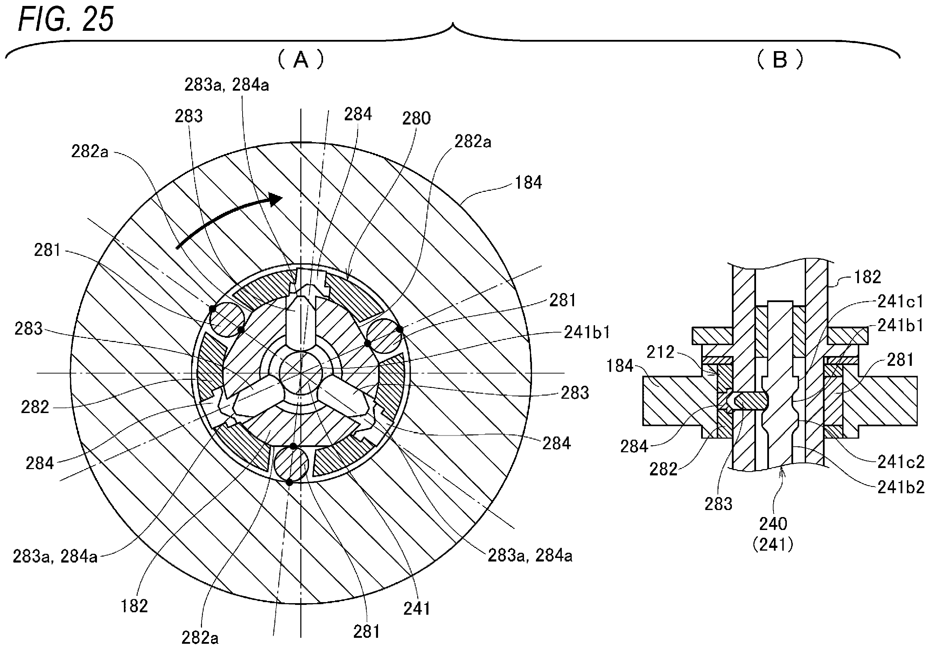

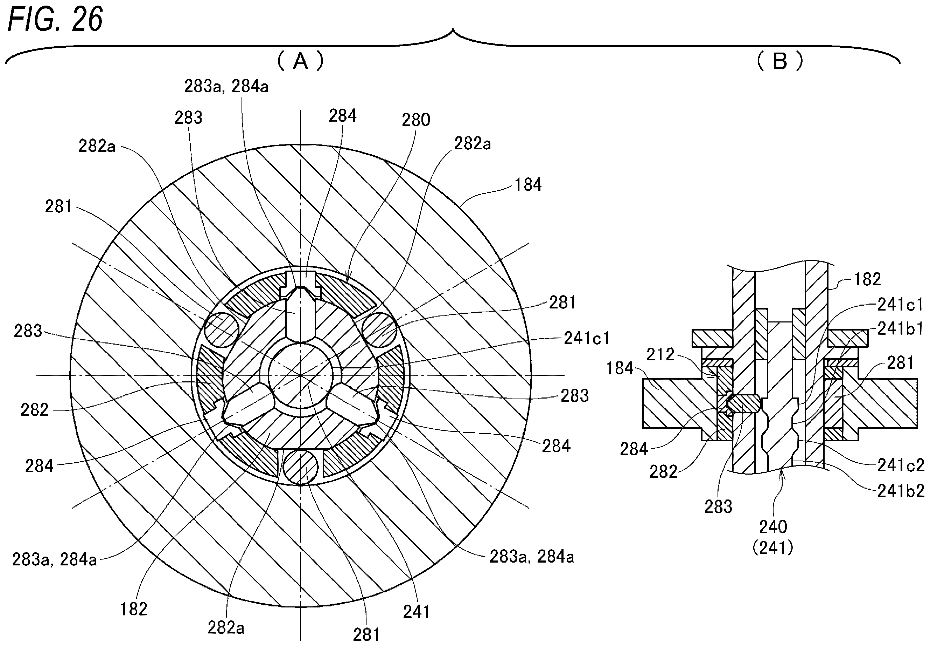

As shown in parts (A) and (B) of Fig. 26, in the ON state shown in parts (A) and (B) of Fig. 25, when the second operation rod 241 moves from the position where the first small diameter portion 241b1 allows the pin 283 of the second connection and disconnection portion 212 to return radially inward to the position where the first large diameter portion 241c1 pushes the pin 283 radially outward, the convex portion 283a of the pin 283 fits into the concave portion 284a of the guide 284, and the guide action of the pin 283 and the guide 284 fixes the relative rotation position of the retainer 282 with respect to the second shaft 182 in a predetermined position. In this sate, the roller 281 is held at the center of the flat portion 282a in the circumferential direction, and thus the roller 281 does not mesh with the outer peripheral surface of the second shaft 182 and the inner peripheral surface of the first driven gear 184, and the OFF state is entered in which the relative rotation between the second shaft 182 and the first driven gear 184 is allowed.

Although detailed description is omitted, the two-way clutches 280 of the fourth connection and disconnection portion 222 according to the third embodiment and the first connection and disconnection portion 211 and the third connection and disconnection portion 221 according to the second embodiment also operate in the same manner, and the two-way clutches 280 can be in the OFF state, the forward rotation ON state, and the reverse rotation ON state. According to the transmissions T according to the second embodiment and the third embodiment, as described above, compared to the transmission T according to the first embodiment, the number of parts can be reduced, the structure can be simplified, and the cost can be reduced. The transmission T according to the third embodiment includes the second connection and disconnection portion 212 and the fourth connection and disconnection portion 222 on a downstream side when the motor M is on an upstream side and the spindle unit SP is on the downstream side in the power transmission path of the motor M, and thus the number of rotation members that follow when the connection and disconnection portions 212 and 222 are made in the OFF state is reduced, so that the electric prosthetic leg 1 can be operated smoothly.

Next, the electric prosthetic leg 1 according to a fourth embodiment of the present invention will be described with reference to Figs. 27 to 32. However, the same reference numerals as those of the second embodiment are used for configurations common to those of the second embodiment, and the description of the embodiment may be incorporated.

The electric prosthetic leg 1 according to the fourth embodiment mainly different from that according to the third embodiment in a housing structure, disposing of the spindle unit SP, connecting the sleeve 174 of the spindle unit SP to the knee upper side member 120 via a link member 320, disposing of the first transmission mechanism T1 and the second transmission mechanism T2, shapes of the drive gears 183 and 185 and the driven gears 184 and 186, and including an extension assistance mechanism 330 that assists the extension of the knee joint mechanism 130 with a force accumulated when the knee is flexed. Hereinafter, details of the differences will be described below.

As shown in Figs. 27 and 28, a housing 310 of the electric prosthetic leg 1 according to the fourth embodiment is open at a top portion and a rear portion, and includes a box-shaped main frame 311 that constitutes the knee lower side member 110, side covers 312 that covers both left and right side surfaces of the main frame 311, and a detachable rear cover 313 that covers the rear opening of the main frame 311 in an openable and closable manner.

The knee upper side member 120 is provided at the top portion of the main frame 311 via the pivot portion 135, and the leg portion 111 is provided at a lower portion of the main frame 311. The expansion and contraction device 140 is incorporated inside the main frame 311. In the expansion and contraction device 140, a unit case 315 is supported by the main frame 311 via a bracket 316.

As shown in Fig. 29, the transmission T includes the first transmission mechanism T1, the second transmission mechanism T, the first connection and disconnection mechanism 210, and the second connection and disconnection mechanism 220. The transmission T according to the fourth embodiment differs from the transmission T of the second embodiment in that the first transmission mechanism T1 is disposed below the second transmission mechanism T2.

The first transmission mechanism T1 includes the first shaft 181 that is mechanically connected to the output shaft of the motor M, the second shaft 182 that is mechanically connected to the spindle 173 of the spindle unit SP, the first drive gear 183 that is provided on the first shaft 181 so as to allow the relative rotation, and the first driven gear 184 that is provided on the second shaft 182 so as to be integrally rotatable and rotates synchronously with the first drive gear 183.

The second transmission mechanism T2 includes the first shaft 181, the second shaft 182, the second drive gear 185 that is provided on the first shaft 181 so as to allow the relative rotation, and the second driven gear 186 that is provided on the second shaft 182 so as to be integrally rotatable and rotates synchronously with the second drive gear 185.

The first connection and disconnection mechanism 210 includes the first connection and disconnection portion 211 that is provided between the first drive gear 183 and the first shaft 181, and the second connection and disconnection mechanism 220 includes the third connection and disconnection portion 221 that is provided between the second drive gear 185 and the first shaft 181. That is, in the transmission T according to the fourth embodiment, the connection and disconnection portion 211 is provided between the first shaft 181 and the gear 183, the connection and disconnection portion 221 is provided between the first shaft 181 and the gear 185, the connection and disconnection portion 212 is not provided between the second shaft 182 and the gear 184, and the connection and disconnection portion 222 is not provided between the second shaft 182 and the gear 186. Since each of the connection and disconnection portion 211 and 221 is provided with the two-way clutch 280 as in the second embodiment, a detailed description will be omitted.

As shown in Figs. 29 to 32, in the electric prosthetic leg 1 according to the fourth embodiment, the spindle unit SP is disposed in front of the pivot portion 135, the knee joint mechanism 130 is flexed according to the extension motion of the spindle unit SP, and the knee joint mechanism 130 is extended according to the contraction motion of the spindle unit SP. According to such a configuration, during a high torque motion for extending the knee joint mechanism 130 from a flexed state (part (A) → part (B) of Fig. 9), a force in a direction opposite to the direction of gravity acts on the spindle 173 of the spindle unit SP, and thus an increase in a size of a support structure that supports the spindle 173 can be avoided. The case where the knee joint mechanism 130 is extended from the flexed state, in other words, is a case where the angle formed by the knee lower side member 110 and the knee upper side member 120 becomes large.

Regarding the support structure that supports the spindle 173, as shown in Fig. 29, the spindle 173 is integrally connected to the second shaft 182 integrated with the first driven gear 184 and the second driven gear 186, and the second shaft 182 is supported by the unit case 315 via pairs of upper and lower bearings BRG. When the angle formed by the knee upper side member 120 and the knee lower side member 110 increases according to the contraction motion of the spindle unit SP, the force in the direction opposite to the direction of gravity acts on the spindle 173 of the spindle unit SP, so that an increase in the size of the bearings BRG can be prevented.

The spindle 173 of the spindle unit SP receives a tensile load from a sleeve 174 side during the high torque motion in which the knee joint mechanism 130 is extended from the flexed state, and thus buckling deformation of the spindle 173 can be prevented.

The gears 183 to 186 are all helical gears, and when the motor M is power-driven, thrust forces are applied from the drive gears 183 and 185 to the driven gears 184 and 186 The gears 183 to 186 are configured such that the thrust force acts on the spindle 173 in the direction opposite to the direction of gravity, so that an increase in the size of the support structure that supports the spindle 173 can be avoided.

As shown in Fig. 29, in the electric prosthetic leg 1 according to the fourth embodiment, the sleeve 174 of the spindle unit SP is connected to the knee upper side member 120 via the link member 320. Specifically, an upper end of the sleeve 174 is connected to a lower end of the link member 320 via a first pivot portion 321, and an upper end of the link member 320 is connected to the knee upper side member 120 via a second pivot portion 322. Thus, the knee joint mechanism 130 can be flexed and extended according to the extension and contraction of the spindle unit SP without supporting the entire expansion and contraction device 140 in a swingable manner as in the electric prosthetic legs 1 according to the first to third embodiments.

Fig. 30 shows the extension state of the electric prosthetic leg 1 according to the fourth embodiment, Fig. 31 shows a state during the extension of the electric prosthetic leg 1, and Fig. 32 shows a maximum flexion state of the electric prosthetic leg 1. While walking with the electric prosthetic leg 1, the maximum flexion state shown in Fig. 32 will not occur. In Fig. 30, a first stopper 342 attached to a support piece 341 that supports the second pivot portion 322 abuts against a position regulating pin 350, the knee joint mechanism 130 is prevented from flexing in the opposite direction. In Fig. 32, a second stopper 343 attached to the knee upper side member 120 abuts against the position regulating pin 350, and flexing from the maximum flexion state of the knee joint mechanism 130 is further prevented. In Figs. 30 to 32, a reference character B represents a battery that supplies electric power to the motor M.

As shown in Figs. 29 to 32, the extension assistance mechanism 330 is provided between the upper end of the link member 320 and the knee upper side member 120 to assist the extension with the force accumulated when the knee joint mechanism 130 is flexed. The extension assistance mechanism 330 includes a pressing portion 332 that presses the upper end of the link member 320 with a biasing force of a spring 331 (for example, a compression coil spring). A cam portion 323 is formed at the upper end of the link member 320. The cam portion 323 continuously includes a small diameter outer peripheral portion 323a centered on the second pivot portion 322 and a large diameter outer peripheral portion 323b having a long distance from the second pivot portion 322, and a connecting outer peripheral portion 323c that connects the small diameter outer peripheral portion 323a and the large diameter outer peripheral portion 323b without steps.

As shown in Fig. 30, in the extension state of the knee joint mechanism 130 with the spindle unit SP contracted, the pressing portion 332 abuts against the small diameter outer peripheral portion 323a of the cam portion 323. As shown in Fig. 31, when the spindle unit SP extends from the extension state of the knee joint mechanism 130 and the knee joint mechanism 130 is flexed, as an abutting position between the pressing portion 332 and the cam portion 323 moves from the small diameter outer peripheral portion 323a to the large diameter outer peripheral portion 323b, the pressing portion 332 is pushed against the biasing force of the spring 331, and the spring 331 is loaded.

Conversely, when the spindle unit SP contracts from the flexion state of the knee joint mechanism 130 and the knee joint mechanism 130 moves from the flexion state to an extension side, as the abutting position between the pressing portion 332 and the cam portion 323 moves from the large diameter outer peripheral portion 323b to the small diameter outer peripheral portion 323a, an accumulated force of the spring 331 acts via the pressing portion 332 and the link member 320 in a direction of contracting the spindle unit SP. Accordingly, the extension assistance mechanism 330 can assist the extension of the knee joint mechanism 130 with the force accumulated when the knee joint mechanism 130 is flexed.

The configuration of the transmission T according to the fourth embodiment is shown in which the connection and disconnection portion 211 is provided between the first shaft 181 and the gear 183, the connection and disconnection portion 221 is provided between the first shaft 181 and the gear 185, the connection and disconnection portion 212 is not provided between the second shaft 182 and the gear 184, and the connection and disconnection portion 222 is not provided between the second shaft 182 and the gear 186, and similar to the transmission T according to the third embodiment, the configuration of the transmission T according to the fourth embodiment may be such that the connection and disconnection portion 212 is provided between the second shaft 182 and the gear 184, the connection and disconnection portion 222 is provided between the second shaft 182 and the gear 186, the connection and disconnection portion 211 is not provided between the first shaft 181 and the gear 183, and the connection and disconnection portion 221 is not provided between the first shaft 181 and the gear 185.

Although various embodiments have been described above with reference to the drawings, it is needless to say that the present invention is not limited to such an example. It is apparent to those skilled in the art that various changes and modifications can be conceived within the scope of the claims, and it is also understood that such variations and modifications belong to the technical scope of the present invention. Components in the embodiments described above may be combined freely within a range not departing from the spirit of the invention.

For example, the embodiments described above describe the prosthetic leg device (electric prosthetic leg) as one embodiment of the joint device of the present invention, whereas the present invention is not limited thereto, and may be a prosthetic limb device (electric prosthetic limb) applied to an elbow joint, and a wearer may be an animal other than a human or may be a robot. When the present invention is applied to the elbow joint, the knee lower side member 110 of the embodiment described above becomes a distal side, that is, a forearm of the wearer with respect to the knee upper side member 120.

The expansion and contraction device 140, the transmission T, the first connection and disconnection mechanism 210 and the second connection and disconnection mechanism 220 provided at the transmission T, the first operation mechanism 230 and the second operation mechanism 240 that switch the first connection and disconnection mechanism 210 and the second connection and disconnection mechanism 220, and the like of the above embodiments are not limited to an application to a joint device, and may be applied to a drive device for a mobile object such as a vehicle, or may be applied to a drive device for a work machine such as a snow removal machine or a lawn mower.

The present specification describes at least the following matters. Corresponding components in the above embodiment are shown in parentheses, without being limited thereto.
(1) Ajoint device (electric prosthetic leg 1), including:
   a first member (knee lower side member 110);
   a second member (knee upper side member 120);
   a connecting unit (knee joint mechanism 130) connecting the first member and the second member such that an angle formed by the first member and the second member is changeable; and
   an expansion and contraction device (expansion and contraction device 140) configured to change the angle formed by the first member and the second member by expansion and contraction, in which
   the expansion and contraction device includes:
      a power source (motor M); and
      a power transmission unit (transmission T) configured to transmit a power of the power source, and
   the power transmission unit includes:
      a first power transmission path (first transmission mechanism T1) through which the power is transmitted at a first transmission gear ratio; and
      a second power transmission path (second transmission mechanism T2) through which the power is transmitted at a second transmission gear ratio different from the first transmission gear ratio.

   According to (1), the connecting unit can be extended and flexed via the power transmission unit that transmits the power of the power source. Since the power transmission unit includes the two power transmission paths with different transmission gear ratios, the power transmission unit can switch between a motion speed and generation power of extension and a motion speed and generation power of flexion in the connecting unit.
(2) The joint device according to (1), in which
   the expansion and contraction device includes:
   a first connection and disconnection mechanism (first connection and disconnection mechanism 210) configured to switch between connection and disconnection of the power in the first power transmission path; and
   a second connection and disconnection mechanism (second connection and disconnection mechanism 220) configured to switch between connection and disconnection of the power in the second power transmission path.

   According to (2), since the expansion and contraction device includes the first connection and disconnection mechanism configured to switch between connection and disconnection of the power in the first power transmission path; and the second connection and disconnection mechanism configured to switch between connection and disconnection of the power in the second power transmission path, the two power transmission paths are appropriately switched.
(3) The joint device according to (1) or (2), in which
   the first power transmission path includes:
      a first rotation member (first drive gear 183) and a second rotation member (first driven gear 184) which are provided to rotate synchronously with each other; and
         (i) a third rotation member (first shaft 181) which is provided rotatably relative to the first rotation member, and a fourth rotation member (second shaft 182) which is provided rotatably relative to the second rotation member, or
         (ii) a third rotation member (first shaft 181) which is provided rotatably relative to the first rotation member, and a fourth rotation member (second shaft 182) which is provided so as to be integrally rotatable with the second rotation member, or
         (iii) a third rotation member (first shaft 181) which is provided so as to be integrally rotatable with the first rotation member, and a fourth rotation member (second shaft 182) which is provided rotatably relative to the second rotation member, and
   the second power transmission path includes:
      a fifth rotation member (second drive gear 185) and a sixth rotation member (second driven gear 186) which are provided to rotate synchronously with each other; and
      (i) a seventh rotation member (first shaft 181) which is provided rotatably relative to the fifth rotation member, and an eighth rotation member (second shaft 182) which is provided rotatably relative to the sixth rotation member, or
      (ii) a seventh rotation member (first shaft 181) which is provided rotatably relative to the fifth rotation member, and an eighth rotation member (second shaft 182) which is provided so as to be integrally rotatable with the sixth rotation member, or
      (iii) a seventh rotation member (first shaft 181) which is provided so as to be integrally rotatable with the fifth rotation member, and an eighth rotation member (second shaft 182) which is provided rotatably relative to the sixth rotation member.

   According to (3), the first power transmission path and the second power transmission path can be configured by the first to eighth rotation members.
(4) The joint device according to (3), in which
   the third rotation member and the seventh rotation member are mechanically connected to the power source.
   According to (4), the power from the power source is input to the first power transmission path and the second power transmission path. The term of "mechanically connected" includes various connection modes that can transmit the power, and includes, for example, direct mutual connection, fastening, and integral formation, as well as a mode in which the power can be transmitted via another member.
(5) The joint device according to (4), in which
   the third rotation member and the seventh rotation member are provided so as to be integrally rotatable with each other.
   According to (5), the rotation members can be shared and connecting structures of the rotation members can be simplified, and thus the expansion and contraction device can be made compact. The term of "integrally rotatable" may be implemented by the same member, or may be implemented by connecting separate members so as to be integrally rotatable.
(6) The joint device according to any of the (3) to (5), in which
   the expansion and contraction device further includes:
      a motion conversion mechanism (spindle unit SP) which is mechanically connected to the power transmission unit and is configured to convert rotary power output from the power transmission unit into translational motion, and
   the fourth rotation member and the eighth rotation member are mechanically connected to the motion conversion mechanism.

   According to (6), the power from the power source is output to the motion conversion mechanism via the first power transmission path and the second power transmission path.
(7) The joint device according to (6), in which
   the fourth rotation member and the eighth rotation member are provided so as to be integrally rotatable with each other.
   According to (7), the rotation members can be shared and the connecting structures of the rotation members can be simplified, and thus the expansion and contraction device can be made compact.
(8) The joint device according to (6) or (7), in which
   the first power transmission path includes:
      the first rotation member and the second rotation member; and
      (i) the third rotation member which is provided rotatably relative to the first rotation member, and the fourth rotation member which is provided rotatably relative to the second rotation member, or
      (iii) the third rotation member which is provided so as to be integrally rotatable with the first rotation member, and the fourth rotation member which is provided rotatably relative to the second rotation member,
   the second power transmission path includes:
      the fifth rotation member and the sixth rotation member; and
      (i) the seventh rotation member which is provided rotatably relative to the fifth rotation member, and the eighth rotation member which is provided rotatably relative to the sixth rotation member, or
      (iii) the seventh rotation member which is provided so as to be integrally rotatable with the fifth rotation member, and the eighth rotation member which is provided rotatably relative to the sixth rotation member,
   the expansion and contraction device includes:
      a first connection and disconnection mechanism (first connection and disconnection mechanism 210) configured to switch between connection and disconnection of the power in the first power transmission path; and
      a second connection and disconnection mechanism (second connection and disconnection mechanism 220) configured to switch between connection and disconnection of the power in the second power transmission path,
   the first connection and disconnection mechanism includes:
      (i) a first connection and disconnection portion (first connection and disconnection portion 211) which is provided between the first rotation member and the third rotation member, and a second connection and disconnection portion (second connection and disconnection portion 212) which is provided between the second rotation member and the fourth rotation member, or
      (iii) a second connection and disconnection portion (second connection and disconnection portion 212) which is provided between the second rotation member and the fourth rotation member, and
   the second connection and disconnection mechanism includes:
      (i) a third connection and disconnection portion (third connection and disconnection portion 221) which is provided between the fifth rotation member and the seventh rotation member, and a fourth connection and disconnection portion (fourth connection and disconnection portion 222) which is provided between the sixth rotation member and the eighth rotation member, or
      (iii) a fourth connection and disconnection portion (fourth connection and disconnection portion 222) which is provided between the sixth rotation member and the eighth rotation member.

   According to (8), when the power source is on an upstream side and the motion conversion mechanism is on a downstream side, at least the connection and disconnection portions are at the downstream side, and thus when the connection and disconnection portions are in an OFF state, the number of following rotation members is reduced, so that the joint device can be operated smoothly.
(9) The joint device according to (8), in which
   each of the second connection and disconnection portion of the first connection and disconnection mechanism and the fourth connection and disconnection portion of the second connection and disconnection mechanism includes:
      an engager (rollers 271 and retainer 274, and rollers 281 and retainer 282) provided between the second rotation member and the fourth rotation member, or between the sixth rotation member and the eighth rotation member, and
   each of the first connection and disconnection mechanism and the second connection and disconnection mechanism includes:
      an operation unit (pins 272 and retainer 274, and pins 283, retainer 282, guides 284, and second operation rod 241) configured to operate the engager between an engaged state and a non-engaged state.

   According to (9), the operation unit can appropriately switch between the OFF state and an ON state of the second connection and disconnection portion of the first connection and disconnection mechanism and the fourth connection and disconnection portion of the second connection and disconnection mechanism.
(10) The joint device according to (9), in which
   each of the operation unit of the first connection and disconnection mechanism and the operation unit of the second connection and disconnection mechanism includes:
   an actuator (pins 272 and retainer 274, and pins 283, retainer 282, and guides 284) that moves the engager; and
   an operator (second operation rod 241) provided to operate the actuator.

   According to (10), the actuators and the operators can appropriately switch between the OFF state and the ON state of the second connection and disconnection portion of the first connection and disconnection mechanism and the fourth connection and disconnection portion of the second connection and disconnection mechanism.
(11) The joint device according to (10), in which
   the fourth rotation member and the eighth rotation member are formed hollow so as to have a common internal space (second internal space S2) extending in a rotation axis direction, and
   the operator of the first connection and disconnection mechanism and the operator of the second connection and disconnection mechanism are disposed in the internal space.

   According to (11), the first connection and disconnection mechanism and the second connection and disconnection mechanism can be made compact.
(12) The joint device according to (11), in which
   the operator of the first connection and disconnection mechanism and the operator of the second connection and disconnection mechanism are integrally formed, and
   the expansion and contraction device further includes a drive unit (second servomotor 242) configured to drive the operators formed integrally.

   According to (12), only one drive unit is required, and thus the expansion and contraction device can be made compact.
(13) The joint device according to any of the (10) to (12), in which
   each of the actuator of the first connection and disconnection mechanism and the actuator of the second connection and disconnection mechanism includes:
      an advancing and retreating element (pins 272 and 283) provided so as to be movable forward and backward along a diameter direction with respect to each of rotation axes of the fourth rotation member and the eighth rotation member,
   each of the operator of the first connection and disconnection mechanism and the operator of the second connection and disconnection mechanism includes:
      an extension portion (large diameter portion 241c) which extends along the rotation axis and is provided so as to be movable forward and backward along the rotation axis, and
   each of the operators is provided such that an outer circumference of the extension portion abuts against an end of the advancing and retreating element on the rotation axis side.

   According to (13), the extension portion provided so as to be movable forward and backward along the rotation axis allows the advancing and retreating element to move forward and backward along the diameter direction.
(14) The joint device according to (13), in which
   the extension portion includes:
      a first extension portion (first large diameter portion 241cl, second large diameter portion 241c2, and third large diameter portion 241c3) abutting against the advancing and retreating element of the first connection and disconnection mechanism; and
      a second extension portion (first large diameter portion 241c1, second large diameter portion 241 c2, and third large diameter portion 241c3) abutting against the advancing and retreating element of the second connection and disconnection mechanism, and
   the first extension portion and the second extension portion are disposed to be positioned at different positions in the rotation axis direction.

   According to (14), by moving the operator forward and backward along the rotation axis, the advancing and retreating element of the first connection and disconnection mechanism and the advancing and retreating element of the second connection and disconnection mechanism can be controlled.
(15) The joint device according to (13) or (14), in which
   each of the advancing and retreating element of the first connection and disconnection mechanism and the advancing and retreating element of the second connection and disconnection mechanism is provided such that:
   when the advancing and retreating element is positioned at an outer side in the diameter direction, the engager is in one of the engaged state and the non-engaged state, and
   when the advancing and retreating element is positioned at an inner side in the diameter direction, the engager is in another of the engaged state and the non-engaged state.

   According to (15), the engaged state and the non-engaged state can be controlled according to the position of the advancing and retreating element in the diameter direction.
(16) The joint device according to (15), in which
   when the operator is positioned at a first position (lower position) in the rotation axis direction, the advancing and retreating element of the first connection and disconnection mechanism is positioned on the outer side in the diameter direction, and the advancing and retreating element of the second connection and disconnection mechanism is positioned at the inner side in the diameter direction, and
   when the operator is positioned at a second position (middle position) in the rotation axis direction, the advancing and retreating element of the first connection and disconnection mechanism is positioned at the inner side in the diameter direction, and the advancing and retreating element of the second connection and disconnection mechanism is positioned at the outer side in the diameter direction.

   According to (16), by moving the operator forward and backward along the rotation axis to the first position or the second position, the first power transmission path and the second power transmission path can be prevented from being able to transmit the power at the same time.
(17) The joint device according to (16), in which
   when each of the advancing and retreating element of the first connection and disconnection mechanism and the advancing and retreating element of the second connection and disconnection mechanism is positioned at the outer side in the diameter direction, the engager is in the non-engaged state, and
   when the extension portion is positioned at a third position (upper position) different from the first position and the second position in the rotation axis direction, the advancing and retreating element of the first connection and disconnection mechanism is positioned at the outer side in the diameter direction, and the advancing and retreating element of the second connection and disconnection mechanism is positioned at the outer side in the diameter direction.

   According to (17), by moving the operator to the third position along the rotation axis, the first power transmission path and the second power transmission path cannot transmit the power at the same time.
(18) The joint device according to any of the (13) to (17), in which
   each of the engager of the first connection and disconnection mechanism and the engager of the second connection and disconnection mechanism includes:
      a plurality of engaging members (rollers 271 and rollers 281) which are disposed to be spaced apart in a circumferential direction with respect to the rotation axis of the fourth rotation member and the eighth rotation member, and
   each of the actuator of the first connection and disconnection mechanism and the actuator of the second connection and disconnection mechanism includes:
      a plurality of the advancing and retreating elements (pins 272 and pins 283) which are disposed to be spaced apart in the circumferential direction with respect to the rotation axis and move the plurality of engaging members; and
      a retainer (retainer 274 and retainer 282) which retains the plurality of engaging members and the plurality of advancing and retreating elements.

   According to (18), the actuator includes the plurality of advancing and retreating elements and the retainer.
(19) The joint device according to (18), in which
   each of the first connection and disconnection mechanism and the second connection and disconnection mechanism includes:
   an interposed member (rubber bulb 282c and O-ring 282d) interposed between the retainer and the second rotation member or between the retainer and the sixth rotation member.
   According to (19), the second rotation member and the fourth rotation member, or the sixth rotation member and the eighth rotation member can be assisted from being in the non-engaged state to the engaged state.
(20) The joint device according to any of the (13) to (17), in which
   each of the engager of the first connection and disconnection mechanism and the engager of the second connection and disconnection mechanism includes:
      a plurality of engaging members (rollers 271 and rollers 281) which are disposed to be spaced apart in a circumferential direction with respect to the rotation axis of the fourth rotation member and the eighth rotation member; and
      a retainer (retainer 274 and retainer 282) which retains the plurality of engaging members, and
   each of the advancing and retreating element of the first connection and disconnection mechanism and the advancing and retreating element of the second connection and disconnection mechanism is provided so as to move the plurality of engagers via the retainer.

   According to (20), the engager includes the plurality of engaging members and the retainer.
(21) The joint device according to any of the (1) to (20), in which
   the joint device is a prosthetic limb device that is attached to a wearer such that the first member is located on a distal side of the wearer with respect to the second member.
   According to (21), the joint device can be used as a prosthetic limb device.
(22) The joint device according to (21), in which
   the prosthetic limb device is a prosthetic leg device attached to a leg portion of the wearer.
   According to (22), the joint device can be used as a prosthetic leg device.
(23) The joint device according to (22), in which
   the second member is attached to a thigh of the leg portion, and
   the connecting unit is provided to function as a knee joint between the thigh and a crus.

   According to (23), the joint device can be used as a knee joint.
(24) The joint device according to (3) or any of (4) to (23) depending on (3), in which
   the joint device is a prosthetic limb device attached to a wearer such that the first member is located on a distal side of the wearer with respect to the second member,
   the prosthetic limb device is a prosthetic leg device attached to a leg portion of the wearer, and
   the first rotation member, the second rotation member, the fifth rotation member, and the sixth rotation member are disposed such that rotation axes of the first rotation member, the second rotation member, the fifth rotation member, and the sixth rotation member extend vertically when the wearer is in an upright state.

   According to (24), the rotation axes of the first rotation member, the second rotation member, the fifth rotation member, and the sixth rotation member can be aligned.
(25) The joint device according to (24), in which
   the expansion and contraction device further includes:

      a motion conversion mechanism (spindle unit SP) which is mechanically connected to the power transmission unit and is configured to convert rotary power output from the power transmission unit into translational motion,
   the motion conversion mechanism includes:
      a shaft member (spindle 173); and
      a cylindrical member (sleeve 174) that performs translational motion along a central axis of the shaft member by rotation of the shaft member,
   the third rotation member and the seventh rotation member are mechanically connected to the power source,
   the fourth rotation member and the eighth rotation member are mechanically connected to the shaft member of the motion conversion mechanism, and
   assuming that out of an acute angle and an obtuse angle, the angle is the acute angle, the motion conversion mechanism applies force in a direction opposite to a direction of gravity to the shaft member when the angle increases.

   According to (25), an increase in a size of a support structure that supports the shaft member of the motion conversion mechanism can be avoided.
(26) The joint device according to (24) or (25), in which
   the expansion and contraction device further includes:

      a motion conversion mechanism (spindle unit SP) which is mechanically connected to the power transmission unit and is configured to convert rotary power output from the power transmission unit into a translational motion,
   the motion conversion mechanism includes:
      a shaft member (spindle 173); and
      a cylindrical member (sleeve 174) that performs translational motion along a central axis of the shaft member by rotation of the shaft member,
   the third rotation member and the seventh rotation member are mechanically connected to the power source,
   the fourth rotation member and the eighth rotation member are mechanically connected to the shaft member of the motion conversion mechanism, and
   assuming that out of an acute angle and an obtuse angle, the angle is the acute angle, the first rotation member and the second rotation member are configured such that when the formed angle becomes larger, a thrust force acting on the second rotation member from the first rotation member acts on the shaft member in a direction opposite to a direction of gravity.

   According to (26), the increase in the size of the support structure that supports the shaft member of the motion conversion mechanism can be avoided.
(27) The joint device according to (26), in which
   the fifth rotation member and the sixth rotation member are configured such that when the formed angle becomes larger, a thrust force acting on the sixth rotation member from the fifth rotation member acts on the shaft member in the direction opposite to the direction of gravity.
   According to (27), the increase in the size of the support structure that supports the shaft member of the motion conversion mechanism can be further avoided.

The present application is based on a Japanese Patent Application (No. 2020-102714) filed on June 12, 2020, the contents of which are incorporated herein by reference.

### REFERENCE SIGNS LIST

1: electric prosthetic leg (joint device)
110: knee lower side member (first member)
120: knee upper side member (second member)
130: knee joint mechanism (connecting unit)
140: expansion and contraction device
173: spindle (shaft member)
174: sleeve (cylindrical member)
181: first shaft (third rotation member, seventh rotation member)
182: second shaft (fourth rotation member, eighth rotation member)
183: first drive gear (first rotation member)
184: first driven gear (second rotation member)
185: second drive gear (fifth rotation member)
186: second driven gear (sixth rotation member)
210: first connection and disconnection mechanism
211: first connection and disconnection portion
212: second connection and disconnection portion
220: second connection and disconnection mechanism
221: third connection and disconnection portion
222: fourth connection and disconnection portion
241: second operation rod (operation unit, operator)
241c: large diameter portion (extension portion)
241c1: first large diameter portion (first extension portion, second extension portion)
241c2: second large diameter portion (first extension portion, second extension portion)
241c3: third large diameter portion (first extension portion, second extension portion)
242: second servomotor (drive unit)
271 roller (engager, engaging member)
272: pin (operation unit, actuator, advancing and retreating element)
274: retainer (operation unit, actuator, engager)
281 roller (engager, engaging member)
282: retainer (operation unit, actuator, engager)
282c: rubber bulb (interposed member)
282d: O-ring (interposed member)
283: pin (operation unit, actuator, advancing and retreating element)
284: guide (operation unit, actuator)
S2: second internal space
T: transmission
T1: first transmission mechanism
T2: second transmission mechanism
SP: spindle unit (motion conversion mechanism)

## Claims

1. A joint device, comprising:
a first member;
a second member;
a connecting unit connecting the first member and the second member such that an angle formed by the first member and the second member is changeable; and
an expansion and contraction device configured to change the angle formed by the first member and the second member by expansion and contraction, wherein
the expansion and contraction device includes:
a power source; and
a power transmission unit configured to transmit power of the power source, and
the power transmission unit includes:
a first power transmission path through which the power is transmitted at a first transmission gear ratio; and
a second power transmission path through which the power is transmitted at a second transmission gear ratio different from the first transmission gear ratio.

2. The joint device according to claim 1, wherein
the expansion and contraction device includes:
a first connection and disconnection mechanism configured to switch between connection and disconnection of the power in the first power transmission path; and
a second connection and disconnection mechanism configured to switch between connection and disconnection of the power in the second power transmission path.

3. The joint device according to claim 1 or 2, wherein
the first power transmission path includes:
a first rotation member and a second rotation member which are provided to rotate synchronously with each other; and
(i) a third rotation member which is provided rotatably relative to the first rotation member, and a fourth rotation member which is provided rotatably relative to the second rotation member, or
(ii) a third rotation member which is provided rotatably relative to the first rotation member, and a fourth rotation member which is provided so as to be integrally rotatable with the second rotation member, or
(iii) a third rotation member which is provided so as to be integrally rotatable with the first rotation member, and a fourth rotation member which is provided rotatably relative to the second rotation member, and
the second power transmission path includes:
a fifth rotation member and a sixth rotation member which are provided to rotate synchronously with each other; and
(i) a seventh rotation member which is provided rotatably relative to the fifth rotation member, and an eighth rotation member which is provided rotatably relative to the sixth rotation member, or
(ii) a seventh rotation member which is provided rotatably relative to the fifth rotation member, and an eighth rotation member which is provided so as to be integrally rotatable with the sixth rotation member, or
(iii) a seventh rotation member which is provided so as to be integrally rotatable with the fifth rotation member, and an eighth rotation member which is provided rotatably relative to the sixth rotation member.

4. The joint device according to claim 3, wherein
the third rotation member and the seventh rotation member are mechanically connected to the power source.

5. The joint device according to claim 4, wherein
the third rotation member and the seventh rotation member are provided so as to be integrally rotatable with each other.

6. The joint device according to any one of claims 3 to 5, wherein
the expansion and contraction device further includes:
a motion conversion mechanism which is mechanically connected to the power transmission unit and is configured to convert rotary power output from the power transmission unit into translational motion, and
the fourth rotation member and the eighth rotation member are mechanically connected to the motion conversion mechanism.

7. The joint device according to claim 6, wherein
the fourth rotation member and the eighth rotation member are provided so as to be integrally rotatable with each other.

8. The joint device according to claim 6 or 7, wherein
the first power transmission path includes:
the first rotation member and the second rotation member; and
(i) the third rotation member which is provided rotatably relative to the first rotation member, and the fourth rotation member which is provided rotatably relative to the second rotation member, or
(iii) the third rotation member which is provided so as to be integrally rotatable with the first rotation member, and the fourth rotation member which is provided rotatably relative to the second rotation member,
the second power transmission path includes:
the fifth rotation member and the sixth rotation member; and
(i) the seventh rotation member which is provided rotatably relative to the fifth rotation member, and the eighth rotation member which is provided rotatably relative to the sixth rotation member, or
(iii) the seventh rotation member which is provided so as to be integrally rotatable with the fifth rotation member, and the eighth rotation member which is provided rotatably relative to the sixth rotation member,
the expansion and contraction device includes:
a first connection and disconnection mechanism configured to switch between connection and disconnection of the power in the first power transmission path; and
a second connection and disconnection mechanism configured to switch between connection and disconnection of the power in the second power transmission path,
the first connection and disconnection mechanism includes:
(i) a first connection and disconnection portion which is provided between the first rotation member and the third rotation member, and a second connection and disconnection portion which is provided between the second rotation member and the fourth rotation member, or
(iii) a second connection and disconnection portion which is provided between the second rotation member and the fourth rotation member, and
the second connection and disconnection mechanism includes:
(i) a third connection and disconnection portion which is provided between the fifth rotation member and the seventh rotation member, and a fourth connection and disconnection portion which is provided between the sixth rotation member and the eighth rotation member, or
(iii) a fourth connection and disconnection portion which is provided between the sixth rotation member and the eighth rotation member.

9. The joint device according to claim 8, wherein
each of the second connection and disconnection portion of the first connection and disconnection mechanism and the fourth connection and disconnection portion of the second connection and disconnection mechanism includes:
an engager provided between the second rotation member and the fourth rotation member, or between the sixth rotation member and the eighth rotation member, and
each of the first connection and disconnection mechanism and the second connection and disconnection mechanism includes an operation unit configured to operate the engager between an engaged state and a non-engaged state.

10. The joint device according to claim 9, wherein
each of the operation unit of the first connection and disconnection mechanism and the operation unit of the second connection and disconnection mechanism includes:
an actuator that moves the engager; and
an operator provided to operate the actuator.

11. The joint device according to claim 10, wherein
the fourth rotation member and the eighth rotation member are formed hollow so as to have a common internal space extending in a rotation axis direction, and
the operator of the first connection and disconnection mechanism and the operator of the second connection and disconnection mechanism are disposed in the internal space.

12. The joint device according to claim 11, wherein
the operator of the first connection and disconnection mechanism and the operator of the second connection and disconnection mechanism are integrally formed, and
the expansion and contraction device further includes a drive unit configured to drive the operators formed integrally.

13. The joint device according to any one of claims 10 to 12, wherein
each of the actuator of the first connection and disconnection mechanism and the actuator of the second connection and disconnection mechanism includes:
an advancing and retreating element provided so as to be movable forward and backward along a diameter direction with respect to a rotation axis of the fourth rotation member and the eighth rotation member,
each of the operator of the first connection and disconnection mechanism and the operator of the second connection and disconnection mechanism includes:
an extension portion which extends along the rotation axis and is provided so as to be movable forward and backward along the rotation axis, and
each of the operators is provided such that an outer circumference of the extension portion abuts against an end of the advancing and retreating element on the rotation axis side.

14. The joint device according to claim 13, wherein
the extension portion includes:
a first extension portion abutting against the advancing and retreating element of the first connection and disconnection mechanism; and
a second extension portion abutting against the advancing and retreating element of the second connection and disconnection mechanism, and
the first extension portion and the second extension portion are disposed to be positioned at different positions in the rotation axis direction.

15. The joint device according to claim 13 or 14, wherein
each of the advancing and retreating element of the first connection and disconnection mechanism and the advancing and retreating element of the second connection and disconnection mechanism is provided such that:
when the advancing and retreating element is positioned at an outer side in the diameter direction, the engager is in one of the engaged state and the non-engaged state, and
when the advancing and retreating element is positioned at an inner side in the diameter direction, the engager is in another of the engaged state and the non-engaged state.

16. The joint device according to claim 15, wherein
when the operator is positioned at a first position in the rotation axis direction, the advancing and retreating element of the first connection and disconnection mechanism is positioned at the outer side in the diameter direction, and the advancing and retreating element of the second connection and disconnection mechanism is positioned at the inner side in the diameter direction, and
when the operator is positioned at a second position in the rotation axis direction, the advancing and retreating element of the first connection and disconnection mechanism is positioned at the inner side in the diameter direction, and the advancing and retreating element of the second connection and disconnection mechanism is positioned at the outer side in the diameter direction.

17. The joint device according to claim 16, wherein
when each of the advancing and retreating element of the first connection and disconnection mechanism and the advancing and retreating element of the second connection and disconnection mechanism is positioned at the outer side in the diameter direction, the engager is in the non-engaged state, and
when the extension portion is positioned at a third position different from the first position and the second position in the rotation axis direction, the advancing and retreating element of the first connection and disconnection mechanism is positioned at the outer side in the diameter direction, and the advancing and retreating element of the second connection and disconnection mechanism is positioned at the outer side in the diameter direction.

18. The joint device according to any one of claims 13 to 17, wherein
each of the engager of the first connection and disconnection mechanism and the engager of the second connection and disconnection mechanism includes:
a plurality of engaging members which are disposed to be spaced apart in a circumferential direction with respect to the rotation axis of the fourth rotation member and the eighth rotation member, and
each of the actuator of the first connection and disconnection mechanism and the actuator of the second connection and disconnection mechanism includes:
a plurality of the advancing and retreating elements which are disposed to be spaced apart in the circumferential direction with respect to the rotation axis and move the plurality of engaging members; and
a retainer which retains the plurality of engaging members and the plurality of advancing and retreating elements.

19. The joint device according to claim 18, wherein
each of the first connection and disconnection mechanism and the second connection and disconnection mechanism includes:
an interposed member interposed between the retainer and the second rotation member or between the retainer and the sixth rotation member.

20. The joint device according to any one of claims 13 to 17, wherein
each of the engager of the first connection and disconnection mechanism and the engager of the second connection and disconnection mechanism includes:
a plurality of engaging members which are disposed to be spaced apart in a circumferential direction with respect to the rotation axis of the fourth rotation member and the eighth rotation member; and
a retainer which retains the plurality of engaging members, and
each of the advancing and retreating element of the first connection and disconnection mechanism and the advancing and retreating element of the second connection and disconnection mechanism is provided so as to move the plurality of engagers via the retainer.

21. The joint device according to any one of claims 1 to 20, wherein
the joint device is a prosthetic limb device that is attached to a wearer such that the first member is located on a distal side of the wearer with respect to the second member.

22. The joint device according to claim 21, wherein
the prosthetic limb device is a prosthetic leg device attached to a leg portion of the wearer.

23. The joint device according to claim 22, wherein
the second member is attached to a thigh of the leg portion, and
the connecting unit is provided to function as a knee joint between the thigh and a crus.

24. The joint device according to claim 3 or any one of claims 4 to 23 depending from claim 3, wherein
the joint device is a prosthetic limb device attached to a wearer such that the first member is located on a distal side of the wearer with respect to the second member,
the prosthetic limb device is a prosthetic leg device attached to a leg portion of the wearer, and
the first rotation member, the second rotation member, the fifth rotation member, and the sixth rotation member are disposed such that rotation axes of the first rotation member, the second rotation member, the fifth rotation member, and the sixth rotation member extend vertically when the wearer is in an upright state.

25. The joint device according to claim 24, wherein
the expansion and contraction device further includes:
a motion conversion mechanism which is mechanically connected to the power transmission unit and is configured to convert rotary power output from the power transmission unit into translational motion, and
the motion conversion mechanism includes:
a shaft member; and
a cylindrical member that performs translational motion along a central axis of the shaft member by rotation of the shaft member,
the third rotation member and the seventh rotation member are mechanically connected to the power source,
the fourth rotation member and the eighth rotation member are mechanically connected to the shaft member of the motion conversion mechanism, and
assuming that out of an acute angle and an obtuse angle, the angle is the acute angle, the motion conversion mechanism applies force in a direction opposite to a direction of gravity to the shaft member when the angle increases.

26. The joint device according to claim 24 or 25, wherein
the expansion and contraction device further includes:
a motion conversion mechanism which is mechanically connected to the power transmission unit and is configured to convert rotary power output from the power transmission unit into translational motion, and
the motion conversion mechanism includes:
a shaft member; and
a cylindrical member that performs translational motion along a central axis of the shaft member by rotation of the shaft member,
the third rotation member and the seventh rotation member are mechanically connected to the power source,
the fourth rotation member and the eighth rotation member are mechanically connected to the shaft member of the motion conversion mechanism, and
assuming that out of an acute angle and an obtuse angle, the angle is the acute angle, the first rotation member and the second rotation member are configured such that when the formed angle becomeslarger, a thrust force acting on the second rotation member from the first rotation member acts on the shaft member in a direction opposite to a direction of gravity.

27. The joint device according to claim 26, wherein
the fifth rotation member and the sixth rotation member are configured such that when the formed angle becomes larger, a thrust force acting on the sixth rotation member from the fifth rotation member acts on the shaft member in the direction opposite to the direction of gravity.
